Europäisches Patentamt

European Patent Office

Office européen des brevets

(19)

(11) **EP 0 575 350 B1**

(12) **EUROPEAN PATENT SPECIFICATION**

(45) Date of publication and mention
of the grant of the patent:
**11.05.2005 Bulletin 2005/19**

(51) Int Cl.⁷: **C12N 5/02**, C12N 5/00,
C12N 15/00, C12M 3/00,
C12M 3/02, C12M 3/04

(21) Application number: **92904136.6**

(22) Date of filing: **17.12.1991**

(86) International application number:
**PCT/US1991/009173**

(87) International publication number:
**WO 1992/011355 (09.07.1992 Gazette 1992/17)**

(54) **METHODS FOR THE EX-VIVO REPLICATION AND STABLE GENETIC TRANSFORMATION OF HUMAN STEM CELLS**

METHODEN FÜR DIE EX-VIVO REPLIKATION UND STABILE GENETISCHE TRANSFORMATION VON MENSCHLICHEN STAMM-ZELLEN

PROCEDES DE EX VIVO REPLICATION ET TRANSFORMATION STABLE GENETIQUE DES CELLULES SOUCHES HUMAINES

(84) Designated Contracting States:
**AT BE CH DE DK ES FR GB GR IT LI LU MC NL SE**

(30) Priority: **17.12.1990 US 628343**
**29.07.1991 US 737024**
**05.08.1991 US 740590**

(43) Date of publication of application:
**29.12.1993 Bulletin 1993/52**

(60) Divisional application:
**04101846.6 / 1 473 360**

(73) Proprietor: **THE REGENTS OF THE UNIVERSITY OF MICHIGAN**
**Ann Arbor, Michigan 48109-1248 (US)**

(72) Inventors:
• **EMERSON, Stephen, G.**
**Ann Arbor, MI 48104 (US)**
• **CLARKE, Michael, F.**
**Ann Arbor, MI 48103 (US)**
• **PALSSON, Bernhard, O.**
**Ann Arbor, MI 48105 (US)**
• **SCHWARTZ, Richard, M.**
**Ann Arbor, MI 48105 (US)**

(74) Representative: **Audier, Philippe André et al Brevalex,**
**3, rue du Docteur Lancereaux**
**75008 Paris (FR)**

(56) References cited:
CA-A- 2 062 741          US-A- 4 514 499
US-A- 4 963 489

• SYMPOSIUM ON TISSUE ENGINEERING HELD AT THE 19TH ANNUAL UCLA (UNIVERSITY OF CALIFORNIA-LOS ANGELES) SYMPOSIA ON MOLECULAR AND CELLULAR BIOLOGY, KEYSTONE, COLORADO, USA, APRIL 6-12, 1990. J CELL BIOCHEM SUPPL 0 (14 PART E). 1990. 243, XP002029342 PALSSON B: "METABOLIC MODELING DESIGN AND OPERATION OF CONTINUOUS PERFUSION HEMATOPOIETIC CULTURES."
• BIOTECHNOL PROG 7 (1). 1991. 1-8, XP002029343 CALDWELL J ET AL: "INFLUENCE OF MEDIUM EXCHANGE SCHEDULES ON METABOLIC GROWTH AND GM-CSF SECRETION RATES OF GENETICALLY ENGINEERED NIH-3T3 CELLS."
• J CELL PHYSIOL 147 (2). 1991. 344-353, XP002029344 CALDWELL J ET AL: "CULTURE PERFUSION SCHEDULES INFLUENCE THE METABOLIC ACTIVITY AND GRANULOCYTE MACROPHAGE COLONY-STIMULATING FACTOR PRODUCTION RATES OF HUMAN BONE MARROW STROMAL CELLS."
• BLOOD (1991), 78(12), 3155-61, XP000616637 SCHWARTZ, RICHARD M. ET AL: "In vitro myelopoiesis stimulated by rapid medium exchange and supplementation with hematopoietic growth factors"

EP 0 575 350 B1

- PROC NATL ACAD SCI U S A 88 (15). 1991. 6760-6764, XP002029345 SCHWARTZ R M ET AL: "RAPID MEDIUM PERFUSION RATE SIGNIFICANTLY INCREASES THE PRODUCTIVITY AND LONGEVITY OF HUMAN BONE MARROW CULTURES."
- PROCEEDINGS OF THE NATIONAL ACADEMY OF SCIENCES OF USA, vol. 87, no. 1, 1 January 1990, pages 439-443, XP000085779 WILSON J M ET AL: "EXPRESSION OF HUMAN ADENOSINE DEAMINASE IN MICE RECONSTITUTED WITH RETROVIRUS-TRANSDUCED HEMATOPOIETIC STEM CELLS"
- JOURNAL OF CELLULAR BIOCHEMISTRY, vol. 45, no. 3, March 1991, pages 268-272, XP000644357 EMERSON S.G. : "The Construction of High Efficiency Human Bone Marrow Tissue Ex Vivo"
- Immunobiology, Volume 166, issued 1984, BODEKER et al., "Production of five human lymphokines (Granulocyte-Macrophage Colony Stimulating Factor, Interferon gamma, Interleukin-2, Macrophage Cytotoxicity Factor and Macrophage Migration Inhibiting Factor) from ConA stimulated lymphocyte, cultures in bioreactors", pages 12-23, see entire article, especially the Abstract.
- DEXTER et al., "Long Term Bone Marrow Culture", published 1984 by Alan R. Liss, Inc., see page 83.
- Nature, Volume 309, issued 28 June 1984, DEXTER, "The message in the medium", pages 746-749, see chart on page 747.
- Blood, Volume 75, Number 11, issued 01 June 1990, COUTINHO et al., "Effects of Recombinant Human Granulocyte Colony-Stimulating Factor (CSF), Human Granulocyte Macrophage CSF and Gibbon Interleukin -3 on Hematoporeses in Human Long-Term Bone Marrow Culture", pages 2118-2129, see entire article.
- The Canadian Journal of Chemical Engineering, Volume 64, issued August 1986, ADAMSON et al., "Industrial Mammalian Cell Culture", pages 531-539, see entire article.
- Science, Volume 248, issued 15 June 1990, WILSON et al., "Correction of CD18-Deficient lymphocytes by Retrovirus-Medicated Gene Transfer", pages 1413-1416, see page 1415 last column, last paragraph.

**Description**

<u>Technical Field</u>

This invention relates to methods for obtaining ex vivo human stem cell division in culture.

<u>Background Art</u>

All of the circulating blood cells in the normal adult, including erythrocytes, leukocytes, platelets and lymphocytes, originate as precursor cells within the bone marrow. These cells, in turn, derive from very immature cells, called progenitors, which are assayed by their development into contiguous colonies of mature blood cells in 1-3 week cultures in semisolid media such as methylcellulose or agar. Progenitor cells themselves derive from a class of progenitor cells called stem cells. Stem cells have the capacity, upon division, for both self-renewal and differentiation into progenitors. Thus, dividing stem cells generate both additional primitive stem cells and somewhat more differentiated progenitor cells. In addition to the generation of blood cells, Stem cells also may give rise to osteoblasts and osteoclasts, and perhaps cells of other tissues as well. This document describes methods and compositions which permit, for the first time, the successful in vitro culture of human hematopoietic stem cells, which results in their proliferation and differentiation into progenitor cells and more mature blood cells.

In the late 1970s the liquid culture system was developed for growing hematopoietic bone marrow *in vitro.* The cultures are of great potential value both for the analysis of normal and leukemic hematopoiesis and for the experimental manipulation of bone marrow, for, e.g., retroviral-mediated gene transfer. These cultures have allowed a detailed analysis of murine hematopoiesis and have resulted in a detailed understanding of the murine system. In addition, it has made possible retroviral gene transfer into cultured mouse bone marrow cells. This allowed tagging murine hematopoietic cells proving the existence of the multi-potent stem cell and of the study of the various genes in the process of leukemogenesis.

But while it has been possible to transfer retroviral genes into cultured mouse bone marrow cells, this is not yet been possible in cultured human bone marrow cells because, to date, human long-term bone marrow cultures have been limited both in their longevity and in their ability to maintain stem cell survival and their ability to produce progenitor cells over time.

Human liquid bone marrow cultures were initially found to have a limited hematopoietic potential, producing decreasing numbers of progenitor cells and mature blood cells, with cell production ceasing by 6 to 8 weeks. Subsequent modifications of the original system resulted only in modest improvements. A solution to this problem is of incalculable value in that it would permit, e.g., expanding human stem cells and progenitor cells for bone marrow transplantation and for protection from chemotherapy, selecting and manipulating such cells, i.e., for gene transfer, and producing mature human blood cells for transfusion therapy.

Studies of hematopoiesis and in vitro liquid marrow cultures have identified fibroblasts and endothelial cells within adhering layers as central cellular stromal elements. These cells both provide sites of attachment for developing hematopoietic cells and can be induced to secrete hematopoietic growth factors which stimulate progenitor cell proliferation and differentiation. These hematopoietic growth factors include granulocyte colony stimulating factor (G-CSF), granulocyte-macrophage colony stimulating factor (GM-CSF) and interleukin-6 (IL-6).

Cultures of human bone marrow cells on such adherent layers in vitro however have been largely disappointing. Unlike related cultures from other species, such as mouse and tree shrew, human liquid marrow cultures fail to produce significant numbers of either nonadherent hematopoietic precursor cells or clonogenic progenitor cells for over 6 to 8 weeks. And although cultures lasting 3-5 months have been reported, no culture which stably produces progenitor cells from stem cells continuously for more than 4-6 weeks has been reported.

Moreover, nonadherent and progenitor cell production typically declined throughout even the short life of these cultures, so that it is not clear that stem cell survival or proliferation is supported at all by these cultures. Further, when studied in isolation, unstimulated bone marrow stromal cells secrete little if any detectable hematopoietic growth factors (HGFs).

The lack of stable progenitor cell and mature blood cell production in these cultures has led to the belief that they are unable to support continual stem cell renewal and expansion. It has therefore been presumed that the cultures either lack a critical stem cell stimulant(s) and/or contain a novel stem cell inhibitor(s). But while explanations for failure to detect HGFs and uninduced stromal cell cultures have been suggested, the null hypothesis, which combines the failure to detect HGFs and the relative failure of human liquid marrow cultures, would be that the culture systems used *in vitro* do not provide the full range of hematopoietic supportive function of adherent bone marrow stromal cells *in vivo*.

Stem cell and progenitor cell expansion for bone marrow transplantation is a potential application for human long-term bone marrow cultures. Human autologous and allogenic bone marrow transplantation are currently used as therapies for diseases such as leukemia, lymphoma and other life-threatening disorders. For these procedures however, a large amount of donor bone marrow must be removed to insure that there is enough cells for engraftment.

A culture providing stem cell and progenitor cell expansion would reduce the need for large bone marrow donation and would make possible obtaining a small marrow donation and then expanding the number of stem cells and progenitor cells *in vitro* before infusion into the recipient. Also, it is known that a small number of stem cells and progenitor cells circulate in the blood stream. If these stem cells and progenitor cells could be collected by phoresis and expanded, then it would be possible to obtain the required number of stem cells and progenitor cells for transplantation from peripheral blood and eliminate the need for bone marrow donation.

Bone marrow transplantation requires that approximately $1 \times 10^8$ to $2 \times 10^8$ bone marrow mononuclear cells per kilogram of patient weight be infused for engraftment. This requires the bone marrow donation of the same number of cells which is on the order of 70 ml of marrow for a 70 kg donor. While 70 ml is a small fraction of the donors marrow, it requires an intensive donation and significant loss of blood in the donation process. If stem cells and progenitor cells could be expanded ten-fold, the donation procedure would be greatly reduced and possibly involve only collection of stem cells and progenitor cells from peripheral blood and expansion of these stem cells and progenitor cells.

Progenitor cell expansion would also be useful as a supplemental treatment to chemotherapy, and is another application for human long-term bone marrow cultures. The dilemma faced by oncologist is that most chemotherapy agents used to destroy cancer act by killing all cells going through cell division. Bone marrow is one of the most prolific tissues in the body and is therefore often the organ that is initially damaged by chemotherapy drugs. The result is that blood cell production is rapidly destroyed during chemotherapy treatment and chemotherapy must be terminated to allow the hematopoietic system to replenish the blood cell supply before a patient is retreated with chemotherapy. It may take a month or more for the once quiescent stem cells to raise up the white blood cell count to acceptable levels to resume chemotherapy during which case the drop in blood cell count is repeated. Unfortunately, while blood cells are regenerating between chemotherapy treatments, the cancer has time to grow and possibly become more resistant to the chemotherapy drugs due to natural selection.

To shorten the time between chemotherapy treatments, large numbers of progenitor and immature blood cells could be given back to the patient. This would have the effect of greatly reducing the time the patient would have low blood cell counts, thereby allowing more rapid resumption of the chemotherapy treatment. The longer chemotherapy is given and the shorter the duration between treatments, the greater the odds of successfully killing the cancer.

The hematopoietic cells required for progenitor cell expansion may come from either bone marrow withdrawal or peripheral blood collection. Bone marrow harvests would result in collection of approximately $4 \times 10^5$ CFU-GM progenitor cells. Phoresis of 5 liters of peripheral blood would collect approximately $10^5$ CFU-GM although this number could be increased to $10^6$ CFU-GM by prior treatment of the donor with GM-CSF. Rapid recovery of a patient would require transfusion of approximately $1 \times 10^8$ to $5 \times 10^8$ CFU-GM which is 100 to 1,000 times more than obtained by routine bone marrow donation or by peripheral blood donation. Therefore, expansion of bone marrow or peripheral blood to increase the number of CFU-GM 2 to 3 orders of magnitude would significantly affect chemotherapy administration and cancer treatment.

Gene therapy is a rapidly growing field in medicine which is also of inestimable clinical potential. Gene therapy has many potential uses in treating disease and has been reviewed extensively. See, e.g., Boggs, Int. J. Cell Cloning. (1990) 8:80-96, Kohn et al, Cancer Invest. (1989) 7 (2):179-192, Lehn, Bone Marrow Transp. (1990) 5:287-293, and Verma, Scientific Amer. (1990) pp. 68-84. Genetically transformed human stem cells have wide potential application in clinical medicine, as agents of gene therapy.

Gene therapy describes an emerging approach to clinical treatment which has evolved from earlier approaches in medical care. The earliest approaches to medical care, evolving over centuries, included gross surgical procedures and the administration of crude mixtures as medicinal agents. In the past century, biochemical pharmacology has supervened as the major method of medical treatment. Under this paradigm, pure biochemical molecules are delivered to the patient. In general, such pharmacologic agents act either as poisons (such as antimicrobials or cancer chemotherapy agents), physiologic mimetics which stimulate endogenous receptors (e.g., opiates, adrenergic agonists), or physiologic antagonists which block endogenous receptors (e.g. antihypertensives, anaesthetics).

Gene therapy is, by definition, the insertion of genes into cells for the purpose of medicinal therapy. The principle underlying gene therapy is to, rather than deliver doses of pharmacologic molecules, deliver a functional gene whose RNA or protein product will produce the desired biochemical effect in the target cell or tissue. There are several potential advantages of gene therapy over classical biochemical pharmacology. First, inserted genes can produce extremely complex molecules, including RNA and protein, which can be extraordinarily difficult or impossible to administer and deliver themselves. Next, controlled insertion of the desired gene into specific target cells can control the production of gene product to defined tissues. Finally, gene therapy can in principle be permanent within an individual, as the gene will continue to function in the target cells and their progeny.

There are several problems that must therefore be addressed for successful gene therapy. The first is to be able to insert the desired therapeutic gene into the chosen cells. Second, the gene must be adequately expressed in the target cell, resulting in the appropriate levels of gene product. Finally the RNA or protein produced must be properly processed by the target cell so that it is functional, i.e. so that gene therapy actually infers clinical therapy. Several methods of gene insertion into human cells *in vitro* are listed in Table 1.

## Table 1: Comparison of DNA transfer methods.

| Variable | Microinjection | Electroporation | Retrovirus |
|---|---|---|---|
| Efficiency | 10-100% | 0.0001-1% | 1-100% (depends on titer) |
| Effort | High | Low | Intermediate |
| Expense | High | Low | Intermediate |
| Stability | Good | Good | May be inactivated or become infective |
| DNA synthesis | ? | ? | Required |
| Size of DNA input | Not restricted | Not restricted | Limited (≤8 kb) |
| Need extraneous DNA | No | No | Yes |

Other techniques, such as homologous recombination, are being developed as well in many laboratories. Research in gene therapy has been on-going for several years in several types of cells *in vitro*, progressed to animal studies, and has recently entered the first human clinical trial(s).

The hematopoietic system is an ideal choice as a delivery system for gene therapy. Hematopoietic cells are readily accessible, simply by bone marrow aspiration or by peripheral blood mononuclear cell harvest. Once the genetic insertion is accomplished *in vitro* the treated cells can be reinfused intravenously, after which the genetically transformed cells will home to and develop in the bone marrow. Since mature blood cells circulate throughout the body, the genetically modified cells can deliver the specific gene product to any desired tissue.

Most importantly, hematopoietic tissues contain stem cells, which possess extensive (perhaps unlimited) capacities for self-renewal. This implies that if genetic material were stably transduced into these stem cells, then upon reinfusion of the hematopoietic tissue, these altered stem cells can expand and repopulate the marrow with cells that express the new gene. This leads to long-lasting, perhaps lifelong delivery of the desired gene product. Similarly, successful stable gene transfer into stem cells located in other tissues, likewise leads to long-lasting gene product delivery.

Successful hematopoietic stem cell gene therapy has broad application, to both diseases specific to the hematopoietic system and to other organ system diseases. Within the hematopoietic system, both inherited and acquired diseases can be treated by stem cell gene therapy. For example, hemoglobin deficiencies such as $\alpha$ and $\beta$ Thalessemias could be treated by the insertion of the gene coding for the globin $\alpha$ or $\beta$ chain, together with regulatory sequences that confer high level tissue specific erythrocytes (see, Grosveld et al, Cell (1987) 51:975-986). Similarly, sickle cell anemia could be corrected by the genetic insertion of the fetal globin gene into hematopoietic stem cells, as the regulated expression of high levels of fetal hemoglobin are sufficient to prevent sickling in red cells despite the copresence of sickle hemoglobin (see, Sunshine et al, J. Molec. Biol. (1979) 133:435).

Genetic disease of neutrophils caused by functional protein deficiencies, such as leukocyte adhesion deficiency (LAD) or chronic granulomatous disease (CGD) could be treated by the genetic insertion of the gene encoding the defective or absent gene, along with regulatory DNA sequences that confer high level, tissue specific expression into hematopoietic stem cells (see, Wilson et al, Science (1990) 248:1413-1416). Genetic diseases involving platelets, such as von Willebrands' Disease, could be corrected by the genetic insertion of the gene encoding, e.g. von Willebrands' Factor, along with sequences which permit its expression and secretion.

The particular suitability of hematopoietic stem cell gene therapy for the replacement of congenitally deficient gene products is particularly evident in the treatment of lymphocyte immunodeficiency diseases, such as severe combined immunodeficiency due to adenosine deaminase deficiency. Retroviral gene therapy of circulating T cells with the ADA gene has been found to be successful at reducing the clinical immunodeficiency experienced by these patients, but the effects are only temporary because the transfected T lymphocytes have a finite life span *in vivo* (see, Kasid et al, Proc. Nat. Acad. Sci. (USA) (1990) 87:473-477, or Culver et al Proc. Nat. Acad. Sci. (USA), (1991) 88:3155-3159). If, however, the gene could be successfully transfected into hematopoietic stem cells, then all of the T cells which arose from these stem cells would contain and express the ADA gene. Therefore, since the transfected stem cells would persist and proliferate for the life of the patient, the T cell ADA deficiency would be permanently treated by a single gene transfer stem cell treatment (see, Wilson et al, Proc. Natl. Acad. Sci., (U.S.A.) (1990) 87:439-443).

In addition to treating inherited enzymatic abnormalities of the hematopoietic system, stem cell gene therapy could be useful for protecting stem cells and their progeny from toxic exogenous agents such as viruses or chemotherapy. For example, gene transfer of DNA sequences encoding the TAR binding site of the HIV TAT transactivating factor have been shown to protect T cells from spreading infection by the HIV virus (see, Sullenger et al, Cell (1990) 63:601-608). Stable transaction of these sequences into hematopoietic stem cells would result in a pool of T cells, all arising from these stem cells, which were relatively or absolutely resistant to the spread of HIV.

Similarly, successful transfection of the genes encoding the multi-drug resistance gene (MDR) or the methotrexate resistance gene into human bone marrow stem cells would create stem cells which were relatively resistant to the effects of cancer chemotherapy. Following autologous bone marrow transplantation with these genetically manipulated cells, patients would be able to tolerate chemotherapy with the agents to which their stem cells were protected with suffering the profound bone marrow suppression commonly caused by these anti-cancer drugs. This would enable patients to receive more effect doses of cancer chemotherapy with less toxicity.

One can readily envision that hematopoietic stem cell gene therapy will also be useful for acquired hematopoietic disease such as leukemia, lymphoma and aplastic anemia. Once the genetic causes of these diseases is discovered, insertion of a gene whose product either overcomes that of the abnormal gene in the cell or corrects it directly (perhaps by splicing out and replacing the gene) would correct the abnormality.

On a broader level, however, hematopoietic stem cell gene therapy can be useful for the treatment of diseases outside the hematopoietic system as well. Gene transfer of DNA sequences carrying therapeutic soluble proteins could give rise to mature blood cells which permanently secreted the desired amounts of a therapeutic molecule. By way of examples, this approach could be useful for the treatment of, e.g., diabetes mellitus by the insertion of DNA sequences for insulin along with regulatory DNA sequences that controlled the proper expression of the transfected insulin gene, perhaps in response to elevated plasma glucose levels. Systemic hypertension could be treated by genetic insertion of stem cells with DNA sequences encoding secretory peptides which act as competitive inhibitors to angiotensin converting enzyme, to vascular smooth muscle calcium channels, or to adrenergic receptors. Alzheimer's disease could possibly be treated by genetic insertion into stem cells of DNA sequences encoding enzymes which break down amyloid plaques within the central nervous system.

The many applications of gene therapy, particularly via stem cell genetic insertion, are thus well known and have been extensively reviewed (see, Boggs et al, supra, Kohn et al, supra, Lehn, supra, and/or Verma et al, supra). There are indeed increasing examples of some success in achieving therapeutic gene transfer into differentiated human stem cells, as described for example in T lymphocytes (see, Kalsd et al, Proc. Nat. Acad. Sci. (U.S.A.), (1990) 87:473-477, Culver et al, Proc. Nat. Acad. Sci. (U.S.A.) (1991) 88:3155-3159).

Unfortunately, achieving (stable) gene transfer into human stem cells has not been accomplished prior to the present invention. While several groups have demonstrated the feasibility of retroviral mediated gene transfer into human hematopoietic cells, human primitive hematopoietic stem cells have not been successfully transfected. This is in sharp contrast to experiments in the mouse, in which some level of retrovirally mediated gene transfer into hematopoietic stem cells has been possible (see, Wilson et al, Pro. Nat. Acad. Sci. (USA) (1990) 87:439-443).

The major impediment to achieving successful human hematopoietic stem cell gene therapy has been the inability to insert genes into human hematopoietic cells under conditions in which the stem cells are dividing and proliferating. Successful stable gene insertion into a target cell requires that the target cell undergo at least one round of cell division. Thus if stem cells are not dividing in the presence of the desired genetic material, the material will not be stably inserted into the stem cells. Prior to the development of the present invention, no system existed which supported the ex vivo division and proliferation of human stem and no successful genetic transformation of human stem cells has been possible.

The literature which is relevant to the present document also includes U.S. Patent No. 4,721,096 which describes a 3-dimensional system involving stromal cells for the growth of hematopoietic cells. See also references cited therein. Glanville et al, Nature (1981) 292:267-269, describe the mouse metallothionein-I gene. Wona et al, Science (1985) 228:810-815, describe human GM-CSF. Lemischka et al, Cell (1986) 45:917-927, describe retrovirus-mediated gene transfer as a marker for hematopoietic stem cells and the tracking of the fate of these cells after transplantation. Yang et al, Cell (1986) 47:3-10, describe human IL-3. Chen et al, Mol. Cell. Biol. (1987) 7:2745-2752, describe transformation of mammalian cells by plasmid DNA. Greaves et al, Cell (1989) 56:979-986, describe the human $CD_2$ gene. Civin et al, J. Immunol. (1984) 133:1576, describe the CD34 antigen. Martin et al, cell (1990) 63:203-211, describes human S-CSF; Forrester et al, J Cell Science (1984), 70:93-110, (1984), discuss parallel flow chamber. Coulombel et al, J. Clin. Invest., (1986) 75:961, describe the loss of CML cells in static cultures.

There is therefore a considerable need for methods and compositions for the *ex vivo* replication and stable genetic transformation of human stem cells and for the optimization of human hematopoietic progenitor cell cultures, particularly in light of the great potential for stem cell expansion, progenitor cell expansion, and gene therapy offered by these systems. Unfortunately, to date, attempts to achieve such results have been disappointing.

Disclosure of the Invention

Accordingly, it is an object of this invention to provide novel methods, including culture media conditions and reactors, for the *ex vivo* replication and stable genetic transformation of human stem cells.

The present invention is based on the inventors' discovery of novel methods, including culture media conditions and reactors, which provide for ex vivo human stem cell division.

These methods are defined as in annexed claims 1-7. They rely on culturing human stem cells in a liquid culture medium which is replaced, preferably perfused, either continuously or periodically, at a rate of 1 milliliter (ml) of medium per ml of culture per about 24 to about 48 hour period, and removing metabolic products and replenishing depleted nutrients while maintaining the culture under physiologically acceptable conditions. In a particularly preferred embodiment of the present invention, the above medium replacement rate is used in conjunction with the addition of hematopoietic growth factors to the rapidly exchanged culture medium.

The inventors have discovered that the increased medium exchange rate used in accordance with the present invention, with the optional addition of hematopoietic growth factors to the rapidly exchanged culture medium, surprisingly (1) supports cultures in which human stem cells proliferate over extended periods of time of at least 5 months, (2) supports cultures in which human hematopoietic progenitor cells are produced by division and differentiation of human stem cells through extended culture periods of at least 5 months, and (3) stimulates the increased metabolism of and GM-CSF secretion from human stromal cells, including human bone marrow stromal cells. The present invention provides, for the first time, human stem cell survival and proliferation in culture.

The present invention also provides an *ex vivo* culture system which supports the continuous proliferation of human stem cells to allow the successful insertion of genetic material into the human stem cells, resulting in the creation of stably genetically transformed human stem cells. This embodiment can be used for the transfer of any genetic material that can be engineered into a recombinant retrovirus, or any other gene transfer vector that requires cell division. Genetically modified human stem cells produced in this manner can be applied to a wide variety of clinical diseases, as described *supra*.

The invention also provides methods for enhancing the efficiency of genetic transfer into human hematopoietic progenitor cells, together with providing stably genetically transformed human stem cells and/or stably genetically transformed human hematopoietic progenitor cells.

Methods are also provided employing reactors and compositions which allow for the efficient proliferation of hematopoietic cells in culture, particularly cells at an early stage in maturation, including stem cells. The methods employ stromal cells, normally transformed, which provide constitutive or inducible production of growth factors, which cells are physically separated to allow for easy separation of hematopoietic cells. By providing for continuous perfusion, and recycling of cells as appropriate, high densities and yields of viable hematopoietic cells may be achieved. The reactor employs a protein surface for the stromal cells and either the surface or other barrier for maintaining separation of stromal cells and hematopoietic cells.

Brief Description of the Figures

Fig. 1 is a schematic view of a perfusion chamber;

Fig. 2 is a schematic representation and flow diagram of the perfusion medium pathway;

Fig. 3a is a schematic view of a flow chamber for measuring shear stress for separation of cells;

Fig. 3b is a side view of the flow chamber of Fig. 3a;

Fig. 3C is a graph of a shear stress profile for hematopoietic cells;

Figs. 4a and 4b are top and side views of a flow chamber for growing and separating hematopoietic cells; and

Figs 5a and 5b are views of a flow chamber in which barriers are removed sequentially allowing the continued growth of stromal cells.

Best Mode for Carrying Out the Invention

The advantages of the present invention may be observed whenever the present invention is applied to any standard system for liquid human stem cell culture, such as those found in hematopoietic culture(s). By the use of the rapid medium exchange rates used in accordance with the present invention, with the optional addition of supplementary hematopoietic growth factors to the culture, the inventors have surprisingly discovered that one is able to make standard systems for liquid human hematopoietic cultures, which comprise cultures performed in the presence or absence of animal sera or plasmas, including horse, calf, fetal calf, or human serum, perform in a qualitatively superior manner.

Human liquid hematopoietic cultures which may be used in accordance with the invention can be performed at cell densities of from $10^4$ to $10^9$ cells per ml of culture, using standard known medium components such as, for example, IMDM, MEM, DMEM, RPMI 1640, Alpha Medium or McCoy's Medium, which can use combinations of serum albumin, cholesterol and/or lecithin, selenium and inorganic salts. As known, these cultures may be supplemented with corticosteroids, such as hydrocortisone at a concentration of $10^{-4}$ to $10^{-7}$ M, or other corticosteroids at equal potent dose, such as cortisone, dexamethasome or solumedrol. These cultures are typically carried out at a pH which is roughly physiologic, i.e. 6.9 to 7.4. The medium is typically exposed to an oxygen-containing atmosphere which contains from 4 to 20 vol. percent oxygen, preferably 6 to 8 vol. percent oxygen.

Using these standard culture techniques, the cell mass used may be enriched, by any desired amount, such as by up to $10^3$ fold or more, either for stem cell content or for hematopoietic progenitor cell content. Different known methods may be used to achieve this enrichment, corresponding either to a negative selection method or a positive selection method. For example, in accordance with the negative selection method, mature cells are removed using immunological techniques, e.g., labelling non-progenitor, non-stem cells with a panel of mouse anti-human monoclonal antibodies, then removing the mouse antibody-coated cells by adherence to rabbit-anti-mouse Ig-coated plastic dishes. See e.g., Emerson et al, J. Clin. Invest. (1985) 76:1286-1290.

The present invention relies on a fundamental alteration of the conditions of liquid human bone marrow cultures under any of the above conditions; rapid replacement of the nutrient medium. Standard culture schedules call for medium and serum to be exchanged weekly, either as a single exchange performed weekly or a one-half medium and serum exchange performed twice weekly. In accordance with the present invention, the nutrient medium of the culture is replaced, preferably perfused, either continuously or periodically, at a rate of about 1 ml per ml of culture per about 24 to about 48 hour period, for cells cultured at a density of from $2 \times 10^6$ to $1 \times 10^7$ cells per ml. For cell densities of from $1 \times 10^4$ to $2 \times 10^6$ cells per ml the same medium exchange rate may be used. For cell densities higher than $10^7$ cells per ml, the medium exchange rate may be increased proportionally to achieve a constant medium and serum flux per cell per unit time.

Replacement of the nutrient medium in accordance with the invention may be carried out in any manner which will achieve the result of replacing the medium, e.g., by removing an aliquot of spent culture medium and replacing it with a fresh aliquot. The flow of the aliquot being added may be by gravity, by pump, or by any other suitable means. The flow may be in any direction or multiplicity of directions, depending upon the configuration and packing of the culture. Preferably, the new medium is added to the culture in a manner such that it contacts the cell mass. Most preferably, it is added the culture in a manner mimicking *in vivo* perfusion, i.e., it is perfused through at least part of the cell mass and up to the whole cell mass.

Another, optional but important, embodiment of the present invention, resides in the addition of hematopoietic growth factors, including synthetic hematopoietic growth factors, to the rapidly exchanged cultures. In a particularly preferred aspect of this embodiment, the cytokines IL-3 and GM-CSF are both added, together, to the medium at a rate of from 0.1 to 100 ng/ml/day, preferably about 0.5 to 10 ng/ml/day, most preferably 1 to 2 ng/ml/day. Epo may be added to the nutrient medium in an amount of from 0.001 to 10 U/ml/day, preferably 0.05 to 0.15 U/ml/day. Mast cell growth factor (MCF, c-kit ligand, Steel factor), may be added to the medium in an amount of from 1 to 100 ng/ml/day, preferably 10 to 50 ng/ml/day. IL-1 ($\alpha$ or $\beta$) may also be added in an amount of from 10 to 100 units/ml per 3 to 5 day period. Additionally, IL-6, G-CSF, basic fibroblast growth factory IL-7, IL-8, IL-9, IL-10, IL-11, PDGF, or EGF to be added, at a rate of from 1 to 100 ng/ml/day.

The inventors have discovered that when IL-3, GM-CSF and Epo are used as described above one obtains lineage specific development of red blood cells. Alternatively, when IL-3 and GM-CSF, with or without IL-6 or G-CSF, are used, the culture preferentially produce granulocytes. The inventors also observed that with the cultures of the invention T and B lymphocytes are lost over time.

The metabolic product level in the medium is normally maintained within a particular range. Glucose concentration is usually maintained in the range of about 5 to 20 mM. Lactate concentration is usually maintained below 35 mM. Glutamine concentration is generally maintained in the range of from about 1 to 3 mM. Ammonium concentration is usually maintained below about 2.4 mM. These concentrations can be monitored by either periodic or on-line continuous measurements using known methods. see, e.g., Caldwell et al, J. Cell. Physiol. (1991) 147:344-353.

The cells which may be cultured in accordance with the present invention may be any human stem cells or human stem cell-containing cellular mass, including human peripheral blood mononuclear cells, human bone marrow cells, and/or human cord blood cells. Each of these cell masses contains human stem cells. Other cellular masses

EP 0 575 350 B1

containing human stem cells may also be used in accordance with the invention, including any human stem cell found in human bone marrow.

In a preferred embodiment of the invention, the cell culture may be enriched to augment the human stem cell content of the cell mass. Such enrichment may achieved as described above, and, when used in accordance with the invention, provides the first useful means for genetic therapy via gene transfer into human stem cells, including human stem cells present in human bone marrow and human bone marrow stem cells. Stem cells present in human bone marrow are cells obtainable from human bone marrow, peripheral blood, fetal liver, or human cord blood.

Generally, in this embodiment, a packaging cell line infected with a retrovirus, or a supernatant obtained from such a packaging cell line culture, or an other gene transfer vector, is added to human stem cells cultured in accordance with of the invention to obtain stably genetically transformed human stem cells. The present invention provides increased levels of stem cell and human hematopoietic progenitor cell replication, whereas, by contrast, prior cultures provided only for human hematopoietic progenitor cell replication at a decreasing rate (i.e., decaying cultures). The present culture system provides, for the first time, expansion of cells in culture, which is required for retroviral infection of cells. Earlier systems in which retroviral infection was carried out on decaying cultures provided no infection of earlier cells. The present invention, particularly when it is practiced together with an enriched stem cell pool, and even more particularly when it is practiced still further with the use of hematopoietic growth factors, including synthetic growth factors, provides a very effective means for obtaining stem cell infection *in vitro*.

The inventors have discovered that addition of supernatants containing recombinant retroviruses to the cultures results in the introduction of the viruses and the genes they carry into human (hematopoietic) stem cells. The progenitor cells which arise by division and differentiation from these stem cells, and the mature blood cells which arise from further division and differentiation of these progenitor cells, contain the transfected DNA throughout the period of the hematopoietic culture *in vitro*. The inventors have observed that when the retrovirus is only added at the beginning period of the culture, they obtain transfected progenitors and mature blood cells which can arise only from stem cells present, proliferating and stably, genetically transformed in the beginning of the culture, because no retrovirally infected cell can infect an adjacent cell. Progenitor cells and more mature cells containing the desired genetic material have accordingly received the gene from the more primitive stem cells genetically transformed during the initial retroviral infection period.

In a preferred embodiment of this aspect of the invention, human hematopoietic cells, either isolated from bone marrow, peripheral blood, fetal liver, or umbilical cord blood, are first enriched for the presence of stem cells by removing more mature blood cells. This is accomplished by incubating the hematopoietic cells with muring monoclonal antibodies recognizing epitopes on mature blood cells and bone marrow precursor cells but not stem cells, and then removing the labelled cells by immunoadherence to a rabbit-anti-mouse-Ig immunoadsorbent surface. The resultant lineage negative (Lin⁻) cells are then cultured in the presence of a retrovirus or other gene transfer vector in accordance with the invention. Preferably the culture is carried out in the presence of GM-CSF (preferably 1 mg/ml/day) and IL-3 (preferably 1 mg/ml/day) with or without IL-1 (preferably 50 U/ml/4 day period), with or without c-kit ligand (Mast cell growth factor) (preferably 10 ug/ml/day).

The retroviral infection may be performed by either including into the culture medium, supernatants (e.g., 5 to 20% vol/vol) produced by retroviral packaging cell lines infected with recombinant retrovirus, during the first 2 to 21, preferably 10 to 14 days of the culture, or by culturing the Lin⁻ cells directly over the infected retroviral packaging lines themselves, or by both.

Preferably, retroviral supernatants are used, and the period of incubation in the presence of virus is 12 to 16 days. Also preferably, the packaging cell lines are grown to near confluency, the medium exchanged, and the cell lines further incubated for 12 to 15 hours. The medium is then collected and used in the transfection of the human stem cells. However, this protocol is not strictly required and any supernatant produced by a retroviral packaging cell line may be used. Any (known) retroviral packaging cell lines may be used in accordance with the invention and cultured in accordance with any known protocol (see, e.g., Wilson et al, Science (1990) 248:1413-1416 and/or Sullenger et al, Cell (1990) 63:601-608). Illustrative packaging cell lines include NIH 3T3 cells and renal carcinoma cell line 5637.

Any gene which is inserted into a recombinant retrovirus together with suitable promoter and enhancer elements that permit its expression can be incorporated into human stem and hematopoietic progenitor cells. The invention provides for the first time conditions that permit stem cell survival and proliferation in these cultures, permitting the creation of stably transfected, genetically modified human hematopoietic stem cells in these cultures. The terms "stably transformed" and "stably transfected" are used in this text to designate incorporation of exogenous DNA into the human stem cell chromosome(s), made possible by the present invention because it permits exposing dividing human stem cells ex vivo to such exogenous DNA.

In accordance with the present invention one obtains cultures in which human hematopoietic progenitor cells are produced by division and differentiation from human stem cells throughout a culture period of at least five months. That is, one obtains a culture which supports stem cell survival and proliferation in culture.

Data obtained by the inventors indicates that medium perfusion rate is a very significant variable in determining

9

the behavior of *ex vivo* human bone marrow cultures. This data showed that when the medium exchange rate was increased from the traditional once per week Dexter rate to a daily medium exchange rate of 7 volumes per week, a significant effect on *ex vivo* hematopoiesis is obtained. In experiments carried out by the inventors, all cultures displayed a significant loss of cells during the first 3 to 4 weeks. Following this decay, the cultures stabilized and the effect of a medium perfusion rate became more pronounced.

A 3.5 per week medium exchange rate led to the most prolific cultures and also to cultures of greatest longevity in terms of progenitor cell production. Of particular note, during weeks 4 to 10, the biweekly number of nonadherent cells produced was actually stable or increasing.

Over the entire course of the cultures, the cumulative number of cells produced after week 3.5 was almost threefold greater than that which is produced under the traditional Dexter culture protocol. Further, stable production of progenitor cells is maintained until week 18.

Human stromal cells, such as stromal cells found in human bone marrow, may or may not be present in the cultures of the invention. In typical cultures, stromal cells are present in the cell culture in an amount of approximately $10^{-3}$ to $10^{-1}$ (stromal cells/total cells).

In another aspect of the invention, the inventors discovered that the cultures of the invention surprisingly provide increased metabolism and GM-CSF and IL-6 secretion from human bone marrow stromal cells. Whereas no GM-CSF is detected in human bone marrow stromal cells supernatant, rapid medium exchange in accordance with the invention stimulates human bone marrow stromal cells to secrete 300 centograms/ml/day to 200 picograms/ml/day of GM-CSF. Secretion of IL-6 by human bone marrow stromal cells is also increased by rapid medium exchange in accordance with the invention from 1 to 2 ng/ml/day to 2 to 4 ng/ml/day. This increase is observed both when only the rapid medium exchange rate of the invention is used, and when the rapid exchange rate together with the addition of hematopoietic growth factors is used. On the basis of data obtained by the inventors, the effect of the rapid medium exchange rates of the invention on human stromal cell production of cytokines should be observed with human stromal cells in any complex tissue culture system.

Illustratively, the medium used in accordance with the invention may comprise three basic components. The first component is a media component comprised of IMDM, HEM, DMEM, RPMI 1640, Alpha Medium or McCoy's Medium, or an equivalent known culture medium component. The second is a serum component which comprises at least horse serum or human serum and may optionally further comprise fetal calf serum, newborn calf serum, and/or calf serum. The third component is a corticosteroid, such as hydrocortisone, cortisone, dexamethasome, solumedrol, or a combination of these, preferably hydrocortisone.

The compositional make up of various media which can be used are set forth below.

## Iscove's Modified Dulbecco's Media (IMDM)[1,2,3]

| COMPONENT | 380-3440 1X Liquid mg/L | 430-2200 Powder mg/L |
|---|---|---|
| **INORGANIC SALTS:** | | |
| CaCl₂ (anhyd.) | 165.00 | 165.00 |
| KCl | 330.00 | 330.00 |
| KNO₃ | 0.076 | 0.076 |
| MgSO₄ (anhyd.) | 97.67 | 97.67 |
| NaCl | 4505.00 | 4505.00 |
| NaHCO₃ | 3024.00 | — |
| NaH₂PO₄·H₂O | 125.00 | 125.00 |
| Na₂SeO₃·5H₂O | 0.0173 | 0.0173 |
| **OTHER COMPONENTS:** | | |
| D-Glucose | 4500.00 | 4500.00 |
| Phenol red | 15.00 | 15.00 |
| HEPES | 5958.00 | 5958.00 |
| Sodium pyruvate | 110.00 | 110.00 |
| **AMINO ACIDS:** | | |
| L-Alanine | 25.00 | 25.00 |
| L-Asparagine·H₂O | 28.40 | 28.40 |
| L-Arginine·HCl | 84.00 | 84.00 |
| L-Aspartic acid | 30.00 | 30.00 |
| L-Cystine·2HCl | 91.24 | 91.24 |
| L-Glutamic acid | 75.00 | 75.00 |
| L-Glutamine | 584.00 | 584.00 |
| Glycine | 30.00 | 30.00 |
| L-Histidine·HCl·H₂O | 42.00 | 42.00 |
| L-Isoleucine | 105.00 | 105.00 |
| L-Leucine | 105.00 | 105.00 |
| L-Lysine·HCl | 146.00 | 146.00 |
| L-Methionine | 30.00 | 30.00 |
| L-Phenylalanine | 66.00 | 66.00 |
| L-Proline | 40.00 | 40.00 |
| L-Serine | 42.00 | 42.00 |
| L-Threonine | 95.00 | 95.00 |
| L-Tryptophan | 16.00 | 16.00 |
| L-Tyrosine·2Na·2H₂O | 103.79 | 103.79 |
| L-Valine | 94.00 | 94.00 |

| COMPONENT | 380-3440 1X Liquid mg/L | 430-2200 Powder mg/L |
|---|---|---|
| **VITAMINS:** | | |
| Biotin | 0.013 | 0.013 |
| D-Ca pantothenate | 4.00 | 4.00 |
| Choline chloride | 4.00 | 4.00 |
| Folic acid | 4.00 | 4.00 |
| i-Inositol | 7.20 | 7.20 |
| Niacinamide | 4.00 | 4.00 |
| Pyridoxal·HCl | 4.00 | 4.00 |
| Riboflavin | 0.40 | 0.40 |
| Thiamine·HCl | 4.00 | 4.00 |
| Vitamin B₁₂ | 0.013 | 0.013 |

1. Dulbecco, R. and Freeman, G. (1959) Virology 8, 396. Smith, J. D., Freeman, G., Vogt, M., and Dulbecco, R. (1960) Virology 12, 185. Tissue Culture Standards Commitee, In Vitro 6, 2, 93.
2. Iscove, N. N. and Melchers, F., J. Experimental Medicine 147, 923.
3. Values shown are in conformance with Tissue Culture Standards Commitee, In Vitro (1970) 6, 6.
3. Iscove, N. N., personal communication.

## Dulbecco's[1] Modified Eagle Media (D-MEM)

| COMPONENT | 328-1825 1X Liquid mg/L | 300-2320 1X Liquid mg/L | 430-1600 Powder mg/L | 320-1965 1X Liquid mg/L | 360-2430 1X Liquid mg/L | 430-2100 Powder mg/L | 430-2000 Powder mg/L | 430-3000 Powder mg/L | 320-1960 1X Liquid mg/L | 320-1970 1X Liquid mg/L | 320-1995 1X Liquid mg/L | 430-3700 Powder mg/L | 320-1960 1X Liquid mg/L | 430-3000 Powder mg/L |
|---|---|---|---|---|---|---|---|---|---|---|---|---|---|---|
| **INORGANIC SALTS:** | | | | | | | | | | | | | | |
| $CaCl_2$ (anhyd.) | 200.00 | 200.00 | 200.00 | 200.00 | 200.00 | 200.00 | 200.00 | 200.00 | 200.00 | 200.00 | 200.00 | 200.00 | 200.00 | 200.00 |
| $Fe(NO_3)_3 \cdot 9H_2O$ | 0.10 | 0.10 | 0.10 | 0.10 | 0.10 | 0.10 | 0.10 | 0.10 | 0.10 | 0.10 | 0.10 | 0.10 | 0.10 | 0.10 |
| KCl | 400.00 | 400.00 | 400.00 | 400.00 | 400.00 | 400.00 | 400.00 | 400.00 | 400.00 | 400.00 | 400.00 | 400.00 | 400.00 | 400.00 |
| $MgSO_4$ (anhyd.) | — | — | 97.67 | — | — | 97.67 | 97.67 | 97.67 | — | — | — | 97.67 | — | 97.67 |
| $MgSO_4 \cdot 7H_2O$ | 200.00 | 200.00 | — | 200.00 | 200.00 | — | — | — | 200.00 | 200.00 | 200.00 | — | 200.00 | — |
| NaCl | 6400.00 | 4750.00 | 6400.00 | 6400.00 | 4750.00 | 6400.00 | 6400.00 | 6400.00 | 6400.00 | 6400.00 | 6400.00 | 4750.00 | 6400.00 | 6400.00 |
| $NaHCO_3$ | 3700.00 | 3700.00 | — | 3700.00 | 3700.00 | — | — | — | 3700.00 | 3700.00 | 3700.00 | — | 3700.00 | — |
| $NaH_2PO_4 \cdot H_2O$ | 125.00 | 125.00 | 125.00 | 125.00 | 125.00 | 125.00 | 125.00 | 125.00 | 125.00 | 125.00 | 125.00 | 125.00 | 125.00 | 125.00 |
| **OTHER COMPONENTS:** | | | | | | | | | | | | | | |
| D-Glucose | 1000.00 | 1000.00 | 1000.00 | 4500.00 | 4500.00 | 4500.00 | 4500.00 | 4500.00 | 4500.00 | 4500.00 | 4500.00 | 4500.00 | 4500.00 | — |
| Phenol red | 15.00 | 15.00 | 15.00 | 15.00 | 15.00 | 15.00 | 15.00 | — | 15.00 | | 15.00 | 15.00 | 15.00 | — |
| HEPES | — | 5958.00 | — | — | 5958.00 | — | — | — | — | — | — | 5958.00 | — | — |
| Sodium pyruvate | 110.00 | 110.00 | 110.00 | — | — | — | 110.00 | — | — | — | 110.00 | — | — | — |
| **AMINO ACIDS:** | | | | | | | | | | | | | | |
| L-Arginine·HCl | 84.00 | 84.00 | 84.00 | 84.00 | 84.00 | 84.00 | 84.00 | 84.00 | 84.00 | 84.00 | 84.00 | 84.00 | 84.00 | 84.00 |
| L-Cystine | 48.00 | 48.00 | — | 48.00 | 48.00 | — | — | — | 48.00 | 48.00 | 48.00 | — | 48.00 | — |
| L-Cystine·2HCl | — | — | 62.57 | — | — | 62.57 | 62.57 | 62.57 | — | — | — | 62.57 | — | 62.57 |
| L-Glutamine | 584.00 | 584.00 | 584.00 | 584.00 | 584.00 | 584.00 | 584.00 | 584.00 | — | — | 584.00 | 584.00 | 584.00 | — |
| Glycine | 30.00 | 30.00 | 30.00 | 30.00 | 30.00 | 30.00 | 30.00 | 30.00 | 30.00 | 30.00 | 30.00 | 30.00 | 30.00 | 30.00 |
| L-Histidine·HCl·H2O | 42.00 | 42.00 | 42.00 | 42.00 | 42.00 | 42.00 | 42.00 | 42.00 | 42.00 | 42.00 | 42.00 | 42.00 | 42.00 | 42.00 |
| L-Isoleucine | 105.00 | 105.00 | 105.00 | 105.00 | 105.00 | 105.00 | 105.00 | 105.00 | 105.00 | 105.00 | 105.00 | 105.00 | 105.00 | 105.00 |
| L-Leucine | 105.00 | 105.00 | 105.00 | 105.00 | 105.00 | 105.00 | 105.00 | 105.00 | 105.00 | 105.00 | 105.00 | 105.00 | 105.00 | 105.00 |
| L-Lysine·HCl | 146.00 | 146.00 | 146.00 | 146.00 | 146.00 | 146.00 | 146.00 | 146.00 | 146.00 | 146.00 | 146.00 | 146.00 | 146.00 | 146.00 |
| L-Methionine | 30.00 | 30.00 | 30.00 | 30.00 | 30.00 | 30.00 | 30.00 | 30.00 | 30.00 | — | 30.00 | 30.00 | 30.00 | 30.00 |
| L-Phenylalanine | 66.00 | 66.00 | 66.00 | 66.00 | 66.00 | 66.00 | 66.00 | 66.00 | 66.00 | 66.00 | 66.00 | 66.00 | 66.00 | 66.00 |
| L-Serine | 42.00 | 42.00 | 42.00 | 42.00 | 42.00 | 42.00 | 42.00 | 42.00 | 42.00 | 42.00 | 42.00 | 42.00 | 42.00 | 42.00 |
| L-Threonine | 95.00 | 95.00 | 95.00 | 95.00 | 95.00 | 95.00 | 95.00 | 95.00 | 95.00 | 95.00 | 95.00 | 95.00 | 95.00 | 95.00 |
| L-Tryptophan | 16.00 | 16.00 | 16.00 | 16.00 | 16.00 | 16.00 | 16.00 | 16.00 | 16.00 | 16.00 | 16.00 | 16.00 | 16.00 | 16.00 |
| L-Tyrosine | 72.00 | 72.00 | — | 72.00 | 72.00 | — | — | — | 72.00 | 72.00 | 72.00 | — | 72.00 | — |
| L-Tyrosine·2Na·2H2O | — | — | 103.79 | — | — | 103.79 | 103.79 | 103.79 | — | — | — | 103.79 | — | 103.79 |
| L-Valine | 94.00 | 94.00 | 94.00 | 94.00 | 94.00 | 94.00 | 94.00 | 94.00 | 94.00 | 94.00 | 94.00 | 94.00 | 94.00 | 94.00 |
| **VITAMINS:** | | | | | | | | | | | | | | |
| D-Ca pantothenate | 4.00 | 4.00 | 4.00 | 4.00 | 4.00 | 4.00 | 4.00 | 4.00 | 4.00 | 4.00 | 4.00 | 4.00 | 4.00 | 4.00 |
| Choline chloride | 4.00 | 4.00 | 4.00 | 4.00 | 4.00 | 4.00 | 4.00 | 4.00 | 4.00 | 4.00 | 4.00 | 4.00 | 4.00 | 4.00 |
| Folic acid | 4.00 | 4.00 | 4.00 | 4.00 | 4.00 | 4.00 | 4.00 | 4.00 | 4.00 | 4.00 | 4.00 | 4.00 | 4.00 | 4.00 |
| i-Inositol | 7.20 | 7.20 | 7.20 | 7.20 | 7.20 | 7.20 | 7.20 | 7.20 | 7.20 | 7.20 | 7.20 | 7.20 | — | 7.20 |
| Niacinamide | 4.00 | 4.00 | 4.00 | 4.00 | 4.00 | 4.00 | 4.00 | 4.00 | 4.00 | 4.00 | 4.00 | 4.00 | 4.00 | 4.00 |
| Pyridoxal·HCl | 4.00 | 4.00 | 4.00 | 4.00 | 4.00 | 4.00 | 4.00 | 4.00 | 4.00 | 4.00 | 4.00 | 4.00 | 4.00 | 4.00 |
| Riboflavin | 0.40 | 0.40 | 0.40 | 0.40 | 0.40 | 0.40 | 0.40 | 0.40 | 0.40 | 0.40 | 0.40 | 0.40 | 0.40 | 0.40 |
| Thiamine·HCl | 4.00 | 4.00 | 4.00 | 4.00 | 4.00 | 4.00 | 4.00 | 4.00 | 4.00 | 4.00 | 4.00 | 4.00 | 4.00 | 4.00 |

[1] Dulbecco, R. and Freeman, G. (1959) Virology 8, 396. Smith, J.D., Freeman, G., Vogt, M. and Dulbecco, R. (1960) Virology 12, 185. Tissue Culture Standards Committee. In Vitro 4, 2, 93.

[2] Values shown are in conformance with the Tissue Culture Standards Committee. In Vitro (1970) 9:6.

## Minimum Essential Media (MEM)[1]

| COMPONENT | 320-2561[2] 1X Liquid | M10-3000 Powder | 320-2571[3] 1X Liquid | M10-1900 Powder | 320-2570 1X Liquid | 320-1690 1X Liquid | 380-2360 1X Liquid | 330-1430 1X Liquid | M10-1700 Powder | 320-1890 1X Liquid | 320-1096 1X Liquid | M10-2400 Powder | 320-1097 1X Liquid | M10-2500 Powder |
|---|---|---|---|---|---|---|---|---|---|---|---|---|---|---|
| | mg/L | mg/L | mg/L | mg/L | mg/L | mg/L | mg/L | mol/L | mg/L | mg/L | mg/L | mg/L | mg/L | mg/L |
| **INORGANIC SALTS:** | | | | | | | | | | | | | | |
| CaCl₂ (anhyd.) | 200.00 | 200.00 | 200.00 | 200.00 | 200.00 | 200.00 | 200.00 | 2000.00 | 200.00 | 200.00 | 200.00 | 200.00 | 200.00 | 200.00 |
| KCl | 400.00 | 400.00 | 400.00 | 400.00 | 400.00 | 400.00 | 400.00 | 4000.00 | 400.00 | 400.00 | 400.00 | 400.00 | 400.00 | 400.00 |
| MgSO₄ (anhyd.) | — | 97.67 | — | 97.67 | — | — | — | — | 97.67 | — | — | 97.67 | — | 97.67 |
| MgSO₄·7H₂O | 200.00 | — | 200.00 | — | 200.00 | 200.00 | 200.00 | 2000.00 | — | 200.00 | 200.00 | — | 200.00 | — |
| NaCl | 6800.00 | 6800.00 | 6800.00 | 6800.00 | 6800.00 | 6600.00 | 6350.00 | 68000.00 | 6800.00 | 6800.00 | 6800.00 | 6800.00 | 6800.00 | 6800.00 |
| NaHCO₃ | 2200.00 | — | 2200.00 | — | 2200.00 | 2200.00 | 2200.00 | — | — | 2200.00 | 1500.00 | — | 2200.00 | — |
| NaH₂PO₄·H₂O | 140.00 | 140.00 | 140.00 | 140.00 | 140.00 | 140.00 | 140.00 | 1400.00 | 140.00 | 140.00 | 140.00 | 140.00 | — | — |
| **OTHER COMPONENTS:** | | | | | | | | | | | | | | |
| D-Glucose | 1000.00 | 1000.00 | 1000.00 | 1000.00 | 1000.00 | 1000.00 | 1000.00 | 10000.00 | 1000.00 | 1000.00 | 1000.00 | 1000.00 | 1000.00 | 1000.00 |
| HEPES | — | — | — | — | — | — | 5958.00 | — | — | — | — | — | — | — |
| Lipoic acid | 0.20 | 0.20 | 0.20 | 0.20 | — | — | — | — | — | — | — | — | — | — |
| Phenol red | 10.00 | 10.00 | 10.00 | 10.00 | 10.00 | 10.00 | 10.00 | 100.00 | 6.00 | 10.00 | 10.00 | 10.00 | 10.00 | 10.00 |
| Sodium pyruvate | 110.00 | 110.00 | 110.00 | 110.00 | — | — | — | — | — | — | — | — | — | — |
| Sodium succinate | — | — | — | — | — | — | — | 100.00 | — | — | — | — | — | — |
| Succinic acid | — | — | — | — | — | — | 75.00 | — | — | — | — | — | — | — |
| **AMINO ACIDS:** | | | | | | | | | | | | | | |
| L-Alanine | 25.00 | 25.00 | 25.00 | 25.00 | — | — | — | — | — | — | — | — | — | — |
| L-Arginine | 105.00 | — | 105.00 | — | — | — | — | — | — | — | — | — | — | — |
| L-Arginine·HCl | — | 126.64 | — | 126.64 | 126.00 | 126.00 | 126.00 | 1260.00 | 126.00 | 126.00 | 126.00 | 126.00 | 126.00 | 126.00 |
| L-Asparagine·H₂O | 50.00 | 50.00 | 50.00 | 50.00 | — | — | — | — | — | — | — | — | — | — |
| L-Aspartic acid | 30.00 | 30.00 | 30.00 | 30.00 | — | — | — | — | — | — | — | — | — | — |
| L-Cystine | 24.00 | — | 24.00 | — | 24.00 | 24.00 | 24.00 | 240.00 | — | 24.00 | — | — | 24.00 | — |
| L-Cystine·2HCl | — | 31.28 | — | 31.28 | — | — | — | — | 31.00 | — | 31.00 | 31.29 | — | 31.29 |
| L-Cysteine·HCl·H₂O | 100.00 | 100.00 | 100.00 | 100.00 | — | — | — | — | — | — | — | — | — | — |
| L-Glutamic acid | 75.00 | 75.00 | 75.00 | 75.00 | — | — | — | — | — | — | — | — | — | — |
| L-Glutamine | 292.00 | 292.00 | 292.00 | 292.00 | 292.00 | — | — | — | — | — | 292.00 | 292.00 | 292.00 | 292.00 |
| Glycine | 50.00 | 50.00 | 50.00 | 50.00 | — | — | — | — | — | — | — | — | — | — |
| L-Histidine | 31.00 | — | 31.00 | — | — | — | — | — | — | — | — | — | — | — |
| L-Histidine·HCl·H₂O | — | 42.00 | — | 42.00 | 42.00 | 42.00 | 42.00 | 420.00 | 42.00 | 42.00 | 42.00 | 42.00 | 42.00 | 42.00 |
| L-Isoleucine | 52.40 | 52.40 | 52.40 | 52.40 | 52.00 | 52.00 | 52.00 | 520.00 | 52.00 | 52.00 | 52.00 | 52.00 | 52.00 | 52.00 |
| L-Leucine | 52.40 | 52.40 | 52.40 | 52.40 | 52.00 | 52.00 | 52.00 | 520.00 | 52.00 | — | 52.00 | — | 52.00 | 52.00 |
| L-Lysine | 58.00 | — | 58.00 | — | — | — | — | — | — | — | — | — | — | — |
| L-Lysine·HCl | — | 72.50 | — | 72.50 | 72.50 | 72.50 | 72.50 | 725.00 | 72.50 | 72.50 | 72.50 | — | 72.50 | 72.50 |
| L-Methionine | 15.00 | 15.00 | 15.00 | 15.00 | 15.00 | 15.00 | 15.00 | 150.00 | 15.00 | 15.00 | 15.00 | — | 15.00 | 15.00 |
| L-Phenylalanine | 32.00 | 32.00 | 32.00 | 32.00 | 32.00 | 32.00 | 32.00 | 320.00 | 32.00 | 32.00 | 32.00 | 32.00 | 32.00 | 32.00 |
| L-Proline | 40.00 | 40.00 | 40.00 | 40.00 | — | — | — | — | — | — | — | — | — | — |
| L-Serine | 25.00 | 25.00 | 25.00 | 25.00 | — | — | — | — | — | — | — | — | — | — |
| L-Threonine | 48.00 | 48.00 | 48.00 | 48.00 | 48.00 | 48.00 | 48.00 | 480.00 | 48.00 | 48.00 | 48.00 | 48.00 | 48.00 | 48.00 |
| L-Tryptophan | 10.00 | 10.00 | 10.00 | 10.00 | 10.00 | 10.00 | 10.00 | 100.00 | 10.00 | 10.00 | 10.00 | 10.00 | 10.00 | 10.00 |
| L-Tyrosine | 36.00 | — | 36.00 | — | 36.00 | 36.00 | 36.00 | 360.00 | 36.00 | 36.00 | — | — | 36.00 | — |
| L-Tyrosine·2Na·2H₂O | — | 51.90 | — | 51.90 | — | — | — | — | — | — | 51.90 | 51.90 | — | 51.90 |
| D-Valine | — | — | — | — | 92.00 | — | — | — | — | — | — | — | — | — |
| L-Valine | 46.00 | 46.00 | 46.00 | 46.00 | — | 46.00 | 46.00 | 460.00 | 46.00 | 46.00 | 46.00 | 46.00 | 46.00 | 46.00 |

## Minimum Essential Media (MEM)[1]—Continued

| COMPONENT | 320-1561[3] 1X Liquid | 410-3000 Powder | 320-2571[3] 1X Liquid | 410-1960 Powder | 320-2570 1X Liquid | 320-1690 1X Liquid | 320-2360 1X Liquid | 330-1430 10X Liquid | 410-1700 Powder | 320-1890 1X Liquid | 320-1960 1X Liquid | 410-2400 Powder | 320-1970 1X Liquid | 410-2500 Powder |
|---|---|---|---|---|---|---|---|---|---|---|---|---|---|---|
| | mg/L | mg/L | mg/L | mg/L | mg/L | mg/L | mg/L | mg/L | mg/L | mg/L | mg/L | mg/L | mg/L | mg/L |
| **VITAMINS:** | | | | | | | | | | | | | | |
| L-Ascorbic acid | 50.00 | 50.00 | 50.00 | 50.00 | — | — | — | — | — | — | — | — | — | — |
| Biotin | 0.10 | 0.10 | 0.10 | 0.10 | — | — | — | — | — | — | — | — | — | — |
| D-Ca pantothenate | 1.00 | 1.00 | 1.00 | 1.00 | 1.00 | 1.00 | 1.00 | 10.00 | 1.00 | 1.00 | 1.00 | 1.00 | 1.00 | 1.00 |
| Choline bitartrate | — | — | — | — | — | — | — | — | 1.80 | — | — | — | — | — |
| Choline chloride | 1.00 | 1.00 | 1.00 | 1.00 | 1.00 | 1.00 | 1.00 | 10.00 | 1.00 | 1.00 | 1.00 | 1.00 | 1.00 | 1.00 |
| Folic acid | 1.00 | 1.00 | 1.00 | 1.00 | 1.00 | 1.00 | 1.00 | 10.00 | 1.00 | 1.00 | 1.00 | 1.00 | 1.00 | 1.00 |
| i-Inositol | 2.00 | 2.00 | 2.00 | 2.00 | 2.00 | 2.00 | 2.00 | 20.00 | 2.00 | 2.00 | 2.00 | 2.00 | 2.00 | 2.00 |
| Niacinamide | 1.00 | 1.00 | 1.00 | 1.00 | 1.00 | 1.00 | 1.00 | 10.00 | 1.00 | 1.00 | 1.00 | 1.00 | 1.00 | 1.00 |
| Pyridoxal-HCl | 1.00 | 1.00 | 1.00 | 1.00 | 1.00 | 1.00 | 1.00 | 10.00 | 1.00 | 1.00 | 1.00 | 1.00 | 1.00 | 1.00 |
| Riboflavin | 0.10 | 0.10 | 0.10 | 0.10 | 0.10 | 0.10 | 0.10 | 1.00 | 0.10 | 0.10 | 0.10 | 0.10 | 0.10 | 0.10 |
| Thiamine-HCl | 1.00 | 1.00 | 1.00 | 1.00 | 1.00 | 1.00 | 1.00 | 10.00 | 1.00 | 1.00 | 1.00 | 1.00 | 1.00 | 1.00 |
| Vitamin B12 | 1.36 | 1.36 | 1.36 | 1.36 | — | — | — | — | — | — | — | — | — | — |
| **RIBONUCLEOSIDES:** | | | | | | | | | | | | | | |
| Adenosine | — | — | 10.00 | 10.00 | — | — | — | — | — | — | — | — | — | — |
| Cytidine | — | — | 10.00 | 10.00 | — | — | — | — | — | — | — | — | — | — |
| Guanosine | — | — | 10.00 | 10.00 | — | — | — | — | — | — | — | — | — | — |
| Uridine | — | — | 10.00 | 10.00 | — | — | — | — | — | — | — | — | — | — |
| **DEOXYRIBONUCLEOSIDES:** | | | | | | | | | | | | | | |
| 2'-Deoxyadenosine | — | — | 10.00 | 10.00 | — | — | — | — | — | — | — | — | — | — |
| 2'-Deoxycytidine-HCl | — | — | 11.00 | 11.00 | — | — | — | — | — | — | — | — | — | — |
| 2'-Deoxyguanosine | — | — | 10.00 | 10.00 | — | — | — | — | — | — | — | — | — | — |
| Thymidine | — | — | 10.00 | 10.00 | — | — | — | — | — | — | — | — | — | — |

1. Eagle, H. (1959) Science. 130, 432.
2. Nature. New Biology (1971) 230, 310.
3. Original formula lists this component as $NaH_2PO_4 \cdot 2H_2O$.

## RPMI Media 1640[1]

| COMPONENT | 330-1870 1X Liquid mg/L | 330-1875 1X Liquid mg/L | 330-2511 10X Liquid mg/L | 380-3400 1X Liquid mg/L | 430-1800 Powder mg/L | 430-3200 Powder mg/L | 430-3400 Powder mg/L | 320-1815 1X Liquid mg/L | 320-1877 1X Liquid mg/L |
|---|---|---|---|---|---|---|---|---|---|
| **INORGANIC SALTS:** | | | | | | | | | |
| Ca(NO₃)₂·4H₂O | 100.00 | 100.00 | 1000.00 | 100.00 | 100.00 | 100.00 | 100.00 | 100.00 | 100.00 |
| KCl | 400.00 | 400.00 | 4000.00 | 400.00 | 400.00 | 400.00 | 400.00 | 400.00 | 400.00 |
| MgSO₄ (anhyd.) | — | — | — | — | 48.84 | 48.84 | 48.84 | — | — |
| MgSO₄·7H₂O | 100.00 | 100.00 | 1000.00 | 100.00 | — | — | — | 100.00 | 100.00 |
| NaCl | 6000.00 | 6000.00 | 60000.00 | 5300.00 | 6000.00 | 6000.00 | 5850.00 | 6000.00 | 6000.00 |
| NaHCO₃ | 2000.00 | 2000.00 | — | 2000.00 | — | — | — | 2000.00 | 2000.00 |
| Na₂HPO₄ (anhyd.) | — | — | — | — | 800.00 | 800.00 | 800.00 | — | — |
| Na₂HPO₄·7H₂O | 1512.00 | 1512.00 | 15120.00 | 1512.00 | — | — | — | 1512.00 | — |
| **OTHER COMPONENTS:** | | | | | | | | | |
| D-Glucose | 2000.00 | 2000.00 | 20000.00 | 2000.00 | 2000.00 | 2000.00 | 2000.00 | 2000.00 | 2000.00 |
| Glutathione (reduced) | 1.00 | 1.00 | 10.00 | 1.00 | 1.00 | 1.00 | 1.00 | 1.00 | 1.00 |
| HEPES | — | — | — | 5958.00 | — | — | 5957.50 | — | — |
| Phenol red | 5.00 | 5.00 | 50.00 | 5.00 | 5.00 | — | 5.00 | — | 5.00 |
| **AMINO ACIDS:** | | | | | | | | | |
| L-Arginine | 200.00 | 200.00 | 2000.00 | 200.00 | 200.00 | 200.00 | 200.00 | 200.00 | 200.00 |
| L-Asparagine | 50.00 | 50.00 | 500.00 | 50.00 | 50.00 | 50.00 | 50.00 | 50.00 | 50.00 |
| L-Aspartic acid | 20.00 | 20.00 | 200.00 | 20.00 | 20.00 | 20.00 | 20.00 | 20.00 | 20.00 |
| L-Cystine | 50.00 | 50.00 | 500.00 | 50.00 | — | — | — | 50.00 | 50.00 |
| L-Cystine·2HCl | — | — | — | — | 65.15 | 65.15 | 65.15 | — | — |
| L-Glutamic acid | 20.00 | 20.00 | 200.00 | 20.00 | 20.00 | 20.00 | 20.00 | 20.00 | 20.00 |
| L-Glutamine | — | 300.00 | 3000.00 | 300.00 | 300.00 | 300.00 | 300.00 | 300.00 | 300.00 |
| Glycine | 10.00 | 10.00 | 100.00 | 10.00 | 10.00 | 10.00 | 10.00 | 10.00 | 10.00 |
| L-Histidine | 15.00 | 15.00 | 150.00 | 15.00 | 15.00 | 15.00 | 15.00 | 15.00 | 15.00 |
| L-Hydroxyproline | 20.00 | 20.00 | 200.00 | 20.00 | 20.00 | 20.00 | 20.00 | 20.00 | 20.00 |
| L-Isoleucine | 50.00 | 50.00 | 500.00 | 50.00 | 50.00 | 50.00 | 50.00 | 50.00 | 50.00 |
| L-Leucine | 50.00 | 50.00 | 500.00 | 50.00 | 50.00 | 50.00 | 50.00 | 50.00 | 50.00 |
| L-Lysine·HCl | 40.00 | 40.00 | 400.00 | 40.00 | 40.00 | 40.00 | 40.00 | 40.00 | 40.00 |
| L-Methionine | 15.00 | 15.00 | 150.00 | 15.00 | 15.00 | 15.00 | 15.00 | 15.00 | 15.00 |
| L-Phenylalanine | 15.00 | 15.00 | 150.00 | 15.00 | 15.00 | 15.00 | 15.00 | 15.00 | 15.00 |
| L-Proline | 20.00 | 20.00 | 200.00 | 20.00 | 20.00 | 20.00 | 20.00 | 20.00 | 20.00 |
| L-Serine | 30.00 | 30.00 | 300.00 | 30.00 | 30.00 | 30.00 | 30.00 | 30.00 | 30.00 |
| L-Threonine | 20.00 | 20.00 | 200.00 | 20.00 | 20.00 | 20.00 | 20.00 | 20.00 | 20.00 |
| L-Tryptophan | 5.00 | 5.00 | 50.00 | 5.00 | 5.00 | 5.00 | 5.00 | 5.00 | 5.00 |
| L-Tyrosine | 20.00 | 20.00 | 200.00 | 20.00 | — | — | — | 20.00 | 20.00 |
| L-Tyrosine·2Na·2H₂O | — | — | — | — | 28.83 | 28.83 | 28.83 | — | — |
| L-Valine | 20.00 | 20.00 | 200.00 | 20.00 | 20.00 | 20.00 | 20.00 | 20.00 | 20.00 |
| **VITAMINS:** | | | | | | | | | |
| Biotin | 0.20 | 0.20 | 2.00 | 0.20 | 0.20 | 0.20 | 0.20 | 0.20 | 0.20 |
| D-Ca pantothenate | 0.25 | 0.25 | 2.50 | 0.25 | 0.25 | 0.25 | 0.25 | 0.25 | 0.25 |
| Choline chloride | 3.00 | 3.00 | 30.00 | 3.00 | 3.00 | 3.00 | 3.00 | 3.00 | 3.00 |
| Folic acid | 1.00 | 1.00 | 10.00 | 1.00 | 1.00 | 1.00 | 1.00 | 1.00 | 1.00 |
| i-Inositol | 35.00 | 35.00 | 350.00 | 35.00 | 35.00 | 35.00 | 35.00 | 35.00 | 35.00 |
| Niacinamide | 1.00 | 1.00 | 10.00 | 1.00 | 1.00 | 1.00 | 1.00 | 1.00 | 1.00 |
| Para-aminobenzoic acid | 1.00 | 1.00 | 10.00 | 1.00 | 1.00 | 1.00 | 1.00 | 1.00 | 1.00 |
| Pyridoxine·HCl | 1.00 | 1.00 | 10.00 | 1.00 | 1.00 | 1.00 | 1.00 | 1.00 | 1.00 |
| Riboflavin | 0.20 | 0.20 | 2.00 | 0.20 | 0.20 | 0.20 | 0.20 | 0.20 | 0.20 |
| Thiamine·HCl | 1.00 | 1.00 | 10.00 | 1.00 | 1.00 | 1.00 | 1.00 | 1.00 | 1.00 |
| Vitamin B₁₂ | 0.005 | 0.005 | 0.05 | 0.005 | 0.005 | 0.005 | 0.005 | 0.005 | 0.005 |

[1] Moore, G.E., Gerner, R.E. and Franklin, H.A. (1967) *J.A.M.A.* 199. 519.

## McCoy's 5A Media (modified)[1,2,3]

| COMPONENT | 330-6600 1X Liquid mg/L | 390-2330 1X Liquid mg/L | 430-1500 Powder mg/L | 330-6600 1X Liquid mg/L | 320-6601* 1X Liquid mg/L | 520-6610 1X Liquid mg/L | 330-6630 1X Liquid mg/L | 320-6650 1X Liquid mg/L |
|---|---|---|---|---|---|---|---|---|
| **INORGANIC SALTS:** | | | | | | | | |
| CaCl₂ (anhyd.) | 100.00 | 100.00 | 100.00 | — | 140.00 | 100.00 | 100.00 | 100.00 |
| KCl | 400.00 | 400.00 | 400.00 | 400.00 | 400.00 | 400.00 | 400.00 | 400.00 |
| KH₂PO₄ | — | — | — | — | 60.00 | — | — | — |
| MgCl₂·6H₂O | — | — | — | — | 100.00 | — | — | — |
| MgSO₄ (anhyd.) | — | — | 97.67 | — | — | — | — | — |
| MgSO₄·7H₂O | 200.00 | 200.00 | — | 200.00 | 100.00 | 200.00 | 200.00 | 200.00 |
| NaCl | 6460.00 | 5100.00 | 6460.00 | 6460.00 | 8000.00 | 6460.00 | 6460.00 | 6460.00 |
| NaHCO₃ | 2200.00 | 2200.00 | — | 2200.00 | 350.00 | 2200.00 | 2200.00 | 2200.00 |
| NaH₂PO₄·H₂O | 580.00 | 580.00 | 580.00 | 1400.00 | — | 580.00 | 580.00 | 580.00 |
| Na₂HPO₄·7H₂O | — | — | — | | 90.00 | — | | |
| **OTHER COMPONENTS:** | | | | | | | | |
| Bacto-peptone | 600.00 | 600.00 | 600.00 | 600.00 | 600.00 | 600.00 | 600.00 | 600.00 |
| Fetal Bovine Serum | — | — | — | — | — | c | c | c |
| D-Glucose | 3000.00 | 3000.00 | 3000.00 | 3000.00 | 1000.00 | 3000.00 | 3000.00 | 3000.00 |
| Glutathione (reduced) | 0.50 | 0.50 | 0.50 | 0.50 | 0.50 | 0.50 | 0.50 | 0.50 |
| HEPES | — | 5958.00 | — | — | — | — | — | — |
| Phenol red | 10.00 | 10.00 | 10.00 | 10.00 | 10.00 | 10.00 | 10.00 | 10.00 |
| **AMINO ACIDS:** | | | | | | | | |
| L-Alanine | 13.90 | 13.90 | 13.90 | 13.90 | 13.90 | 13.90 | 13.90 | 13.90 |
| L-Arginine·HCl | 42.10 | 42.10 | 42.10 | 42.10 | 42.10 | 42.10 | 42.10 | 42.10 |
| L-Asparagine* | 45.00 | 45.00 | 45.00 | 45.00 | 45.00 | 45.00 | 45.00 | 45.00 |
| L-Aspartic acid | 19.97 | 19.97 | 19.97 | 19.97 | 19.97 | 19.97 | 19.97 | 19.97 |
| L-Cysteine* | 31.50 | 31.50 | 31.50 | 31.50 | 31.50 | 31.50 | 31.50 | 31.50 |
| L-Glutamic acid | 22.10 | 22.10 | 22.10 | 22.10 | 22.10 | 22.10 | 22.10 | 22.10 |
| L-Glutamine | 219.20 | 219.20 | 219.20 | 219.20 | 219.20 | 219.20 | 219.20 | 219.20 |
| Glycine | 7.50 | 7.50 | 7.50 | 7.50 | 7.50 | 7.50 | 7.50 | 7.50 |
| L-Histidine·HCl·H₂O | 20.96 | 20.96 | 20.96 | 20.96 | 20.96 | 20.96 | 20.96 | 20.96 |
| L-Hydroxyproline | 19.70 | 19.70 | 19.70 | 19.70 | 19.70 | 19.70 | 19.70 | 19.70 |
| L-Isoleucine | 39.36 | 39.36 | 39.36 | 39.36 | 39.36 | 39.36 | 39.36 | 39.36 |
| L-Leucine | 39.36 | 39.36 | 39.36 | 39.36 | 39.36 | 39.36 | 39.36 | 39.36 |
| L-Lysine·HCl | 36.50 | 36.50 | 36.50 | 36.50 | 36.50 | 36.50 | 36.50 | 36.50 |
| L-Methionine | 14.90 | 14.90 | 14.90 | 14.90 | 14.90 | 14.90 | 14.90 | 14.90 |
| L-Phenylalanine | 16.50 | 16.50 | 16.50 | 16.50 | 16.50 | 16.50 | 16.50 | 16.50 |
| L-Proline | 17.30 | 17.30 | 17.30 | 17.30 | 17.30 | 17.30 | 17.30 | 17.30 |
| L-Serine | 26.30 | 26.30 | 26.30 | 26.30 | 26.30 | 26.30 | 26.30 | 26.30 |
| L-Threonine | 17.90 | 17.90 | 17.90 | 17.90 | 17.90 | 17.90 | 17.90 | 17.90 |
| L-Tryptophan | 3.10 | 3.10 | 3.10 | 3.10 | 3.10 | 3.10 | 3.10 | 3.10 |
| L-Tyrosine | 18.10 | 18.10 | — | 18.10 | 18.10 | 18.10 | 18.10 | 18.10 |
| L-Tyrosine·2Na·2H₂O | — | — | 26.10 | — | — | — | — | — |
| L-Valine | 17.60 | 17.60 | 17.60 | 17.60 | 17.60 | 17.60 | 17.60 | 17.60 |

## McCoy's 5A Media (modified)[1,2,3]—Continued

| COMPONENT | 320-4400 1X Liquid mg/l | 340-2150 1X Liquid mg/L | 430-1500 Powder mg/L | 320-4450 1X Liquid mg/L | 320-4451 1X Liquid mg/L | 320-6610 1X Liquid mg/L | 320-6620 1X Liquid mg/L | 320-6630 1X Liquid mg/L |
|---|---|---|---|---|---|---|---|---|
| **VITAMINS:** | | | | | | | | |
| Ascorbic acid | 0.50 | 0.50 | 0.50 | 0.50 | 0.50 | 0.50 | 0.50 | 0.50 |
| Biotin | 0.20 | 0.20 | 0.20 | 0.20 | 0.20 | 0.20 | 0.20 | 0.20 |
| Choline chloride | 5.00 | 5.00 | 5.00 | 5.00 | 5.00 | 5.00 | 5.00 | 5.00 |
| D-Ca pantothenate | 0.20 | 0.20 | 0.20 | 0.20 | 0.20 | 0.20 | 0.20 | 0.20 |
| Folic acid | 10.00 | 10.00 | 10.00 | 10.00 | 10.00 | 10.00 | 10.00 | 10.00 |
| i-Inositol | 36.00 | 36.00 | 36.00 | 36.00 | 36.00 | 36.00 | 36.00 | 36.00 |
| Niacinamide | 0.50 | 0.50 | 0.50 | 0.50 | 0.50 | 0.50 | 0.50 | 0.50 |
| Nicotinic acid | 0.50 | 0.50 | 0.50 | 0.50 | 0.50 | 0.50 | 0.50 | 0.50 |
| Para-aminobenzoic acid | 1.00 | 1.00 | 1.00 | 1.00 | 1.00 | 1.00 | 1.00 | 1.00 |
| Pyridoxal-HCl | 0.50 | 0.50 | 0.50 | 0.50 | 0.50 | 0.50 | 0.50 | 0.50 |
| Pyridoxine-HCl | 0.50 | 0.50 | 0.50 | 0.50 | 0.50 | 0.50 | 0.50 | 0.50 |
| Riboflavin | 0.20 | 0.20 | 0.20 | 0.20 | 0.20 | 0.20 | 0.20 | 0.20 |
| Thiamine-HCl | 0.20 | 0.20 | 0.20 | 0.20 | 0.20 | 0.20 | 0.20 | 0.20 |
| Vitamin B12 | 2.00 | 2.00 | 2.00 | 2.00 | 2.00 | 2.00 | 2.00 | 2.00 |

1. McCoy, T.A., Maxwell, M., and Kruse, P.F. (1959) Proc. Soc. Exptl. Biol. Med. 100, 115.
2. Hsu, I.C. and Kellogg, D.S., Jr. (1960) J. Natl. Cancer Inst. 25, 221.
3. Iwakata, S. and Grace, J.I., Jr. (1964) N.Y.J. Med. 64:18, 2279
4. McCoy's 5A Medium formulated with Hanks' and Sunnenburn Salts is a GIBCO modification and is not cited in references 1-3
5. HCl form used by the Tissue Culture Standards Committee. In term (1974) 570
6. Monohydrate form listed by the Tissue Culture Standards Committee. In term (1974) 9-6.
7. Fetal Bovine Serum Supplementation: Cat. No. FBS
   320-6011   10% v/v
   320-6031   20% v/v
   320-6030   30% v/v

The serum component may be present in the culture in an amount of at least 1% (v/v) to 50% (v/v). The serum concentration may be preferably in the neighborhood of 15 to 30% (v/v). For higher serum concentrations, the exchange rate is increased proportionately. The third component may be present in an amount of from $10^{-7}$ M to $10^{-4}$ M, and is preferably present in an amount of from $5 \times 10^{-6}$ to $5 \times 10^{-5}$ M. The media component represents the balance such that all three components add up to 100%. Alternatively the serum component can be replaced by any of several standard serum replacement mixtures which typically include insulin, albumin, and lecithin or cholesterol. See, Migliaccio et al, Exp. Hematol. (1990) 18:1049-1055, Iscove et al, Exp. Cell Res. (1980) 126:121-126, and Dainiak et al, J. Clin. Invest. (1985) 76:1237-1242.

Illustratively, human hematopoietic stem cell concentration may be increased as follows. Red blood cells are removed from a bone marrow aspirate by ficol-hypaque gradient centriguration. The mononuclear cells are then incubated with a "cocktail" of antibodies which recognize mature blood elements including red blood cells and red blood cell, granulocytes, macrophages, and mature lymphocytes (both B- and T-cells). In addition, antibodies are included which recognize committed progenitor cells (including anti CD 33). The mature cells are then removed by one of various procedures including panning, magnetic beads, or cell sorting on a Fluorescent Activated Cell Sorter (FACS). By removing the mature elements, the chance of recombinant virus infection, and therefore transfer of the desired gene(s), to hematopoietic stem cells is greatly facilitated.

In another embodiment, methods are provided for the growth of hematopoietic cells in culture, employing fibroblast cells, normally transformed, for providing growth factors, with proteinaceous components added to the mixtures of the fibroblast cells and hematopoietic cells, and substantially continuous perfusion, optionally with recycling, to maintain an effective growth environment.

The description of the method of this embodiment therefore may be divided into descriptions of the perfusion conditions, the reactor and its internal structure, and the transformed fibroblasts.

The reactor comprises a vessel which may be of any convenient shape which allows for the necessary cell distribution, introduction of nutrients and oxygen, removal of waste metabolic products, optional recycling of hematopoietic cells, substitution of stromal cells, and harvesting of hematopoietic cells. The reactor should provide for conditions which substantially mimic bone perfusion. *In vivo*, about 0.08 ml of serum per ml of bone marrow per minute is perfused. This translates into about 0.3 ml of serum per $10^6$ cells per day. The media will therefore be changed on the average at least 50%, preferably at least 100%, in any 24 hour period, so as to maintain a level of metabolic products which is not growth limiting. The rate of change will generally be from about 0.5 to 1.0 ml of perfusion medium per $10^6$ cells per day, empirically mimicking *in vivo* perfusion rates.

The rate of perfusion in the bioreactor will vary depending on the cell density in the reactor. For cells cultured at $2\text{-}10 \times 10^6$ cells/ml, this rate is 1 ml/ml reactor volume per 24-48 hours, where the medium used contains 20% serum, either 10% fetal calf serum and 10% horse serum, or 20% fetal calf serum. For higher cell densities, the perfusion rate will be increased proportionately to achieve a constant serum flux per cell per time. Thus if the cells are cultured at 5 x $10^8$ cell/ml the perfusion rate will be 0.1 ml/ml reactor volume per minute. These flow rates, matching serum and medium flux rates to cell density, are essential to stimulating the endogenous production of hematopoietic growth factors from the normal human bone marrow stromal cells in the culture. The hematopoietic growth factors induced by these serum and medium flux rates include GM-CSF, and may also include S-CSF, IL-6 and G-CSF as well as other hematopoietic growth factors. These rates will be established in the bioreactors such that the shear stress from longitudinal flow experienced by the stem cells and progenitor cells at their stromal cell attachment sites are between 1.0 and 5.0 dynes/square cm.

Various media may be employed for the growth of hematopoietic and stromal cells. Illustrative media include MEM, IMDM, and RPMI, which may be supplemented by combinations of 5-20% fetal calf serum, 5-20% calf serum, and 0-15% horse serum, and/or serum free media supplemented with PDGF, EGF, FGF, HGF or other growth factors to stimulate stromal cells or stem cells. To supplement the growth factors provided by the transformed fibroblasts, additional growth factors may be included in the perfusion medium, particularly where dedicated cells of a particular lineage are desired. Among the growth factors which may be included in the perfusion medium, either by stromal cell secretion or addition, are GM-CSF, G-CSF, or M-CSF, interleukins 1-7, particularly 1, 3, 6, and 7, TGF-$\alpha$ or $\beta$, erythropoietin, or the like, particularly human factors. Of particular interest is the presence of about 0.5-2, preferably 1, ng/ml G-MCSF, and 0.5-2, preferably 1 ng/ml, as well as a 0.1-2 U/ml/day of final concentration of erythropoietin, from about 100-300 ng/ml/day of G-CSF and about 1-10 ng/ml/day of stem cell growth factor (S-CSF, also referenced as Mast Cell Growth Factor or Kit ligand). It is understood that one or more, preferably at least two of the growth factors will be provided by secretion from transformed cells, which will be present in an amount sufficient to maintain the desired level of the growth factors in the perfusion medium.

Conveniently, in the reactor, physiologic temperature will be employed, namely 37°C, although lower temperatures may also be employed, including 33°, usually not being below 25°C. Humidity will generally be about 100%, where the air will contain about 5% carbon dioxide. The perfusion medium may be oxygenated external to the reactor or internal to the reactor, various means being provided for internal oxygenation. Internal oxygenation may be achieved with hollow fibers, porous sintered disks, silicone tubing or other membranes of suitable porosity and hydrophobicity. The nutrient level and metabolic product level will normally be maintained in a relatively narrow range. Glucose level will usually be in the range of about 5 to 20 mM, usually about 10 to 20 mM, lactate concentration will usually be maintained below about 35 mM and may be allowed to be over 20 mM. Glutamine concentration will generally be maintained in the range of about 1 to 3 mM, usually 1.5 to 2.5 mM, while ammonia concentration will usually be maintained below about 2.5 mM, preferably below about 2.0 mM.

The flow of fluid may be by gravity, by a pump, or other means, where the flow may be in any direction or a multiplicity of directions, depending upon the nature of the packing in the reactor. Desirably, laminar flow may be employed where the flow may be substantially horizontal across the reactor or vertical flow may be employed, where the flow is from the bottom to the top of the reactor or vice-versa.

Where the source of human hematopoietic cells is suspected of having neoplastic cells, e.g., leukemic lymphoma or carcinoma, the perfusion flow can be selected so as to segregate the normal progenitor cells from the neoplastic hematopoietic cells. It is found that normal hematopoietic progenitor cells adhere to stroma and matrix proteins with an affinity able to withstand approximately 1.5-2.0 dynes/cm$^2$ stress from longitudinal fluid flow. By contrast, neoplastic cells and their progenitors have a substantially weaker affinity for stroma, in the range of about 0.05-1.2 dynes/cm$^2$. By providing for a perfusion flow rate which provides sheer stress rates intermediate between that tolerated by normal and neoplastic progenitor cells, generally greater than 1 dyne/cm$^2$, one can provide for separation of the neoplastic progenitor cells from the normal progenitor cells, generally maintaining the perfusion for at least about two days, preferably at least about five days, and more preferably seven days or more. In this manner, one can expand normal hematopoietic cells from a human patient, while at the same time using the appropriate flow rates, separate neoplastic cells. In this manner, one can provide for autologous hematopoietic cells from a patient suffering from neoplasia, expand the normal hematopoietic cells during a period of treatment of the patient by chemotherapy or X-irradiation, and then restore normal hematopoietic cells to the patient to restore hematopoiesis and the immune system of the patient.

Illustrative of the use of shear stress to separate hematopoietic tumor cells from normal hematopoietic cells is the situation of chronic myelogenous leukemia (CML). Shear stress tolerance for CML cells is in the range of 0.05-1.2 dyne/cm$^2$. This difference permits the efficient removal of CML cells with an individual bone marrow sample. By employing a shear rate of about 1.2-1.5, preferably 1.3, dynes/cm$^2$, the CML cell may be efficiently separated.

The shear stress tolerance within an individual's bone marrow cells may be determined using a tapered radial flow chamber. In the radial flow chamber, the shear stress experienced by the cell decreases with distance "d" from the start of the chamber as a function of 1/d. Bands may then be analyzed for cell population and the shear stress set for the desired cell population to be retained.

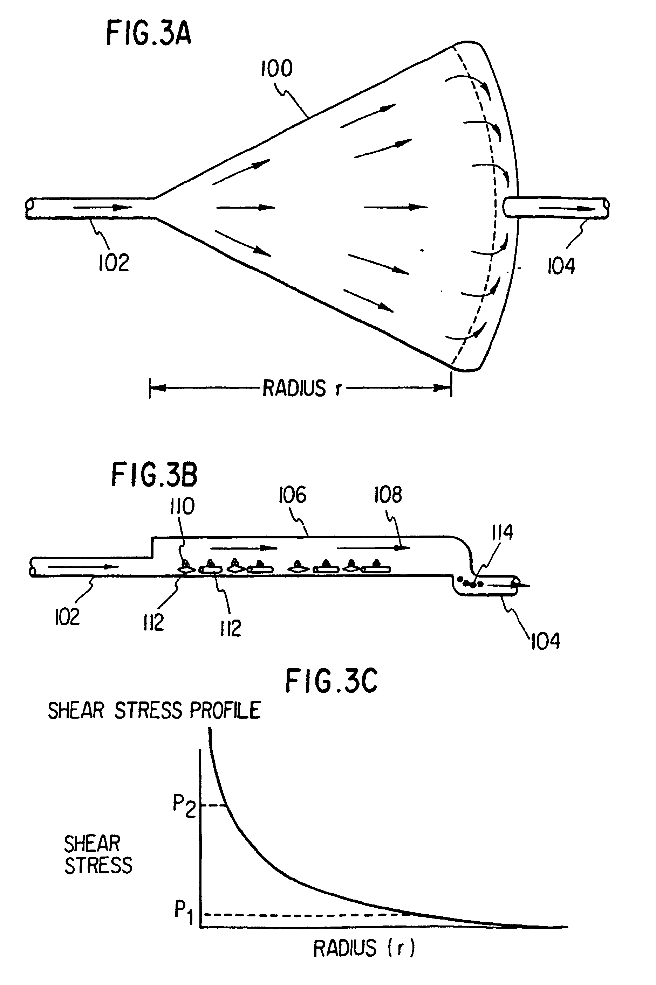

For the removal of leukemic stem cells, progenitor cells and stem cells from bone marrow samples from patients with leukemia are first placed into a radial flow chamber. The radial flow chamber consists of two parallel plates, made of polycarbonate or glass, which permit the adhesion of bone marrow stromal cells to the lower plates. The initial measurements can be performed by either 1) establishing a preformed confluent monolayer of bone marrow stromal cells prior to hematopoietic cell infusion and then initiating fluid flow after 12-24 hours, or 2) inoculating the patient's bone marrow directly into the flow chamber without using a preformed stromal monolayer, and then waiting 3-4 days before establishing the fluid flow, usually 0.05-1.0 cc/min. The plates are sealed together at the edges through a rubber gasket, and held together with adjustable screws. At the narrow, infusion, end of the chamber a tube brings fluid into the chamber from a reservoir delivered by a constant pressure syringe-type pump. At the wide, collection end, the fluid and removed cells are collected through a separate tube (see Figs. 3 and 3b). After the period of perfusion (usually 3-7 days), the nonadherent cells are removed, and the plates are separated, cells from each of 3-5 regions are separately removed by aspiration and rubber policeman, and each fraction is analyzed for the presence of leukemic cells by standard techniques (usually karyotypic analysis by chromosomal banding). Comparison of the leukemic analyses of each fraction demonstrates in which fraction (i.e. at which shear stress), the leukemic cells fail to adhere to the stroma and are removed. In these chambers, the shear stress perceived by the cells declines exponentially as a function of the distance are from the inlet. (See Fig. 3c). Typically, the nonadherent cells are all or nearly all leukemic, whereas cells adhering at the in the narrowest 1/2 of the chamber are all or nearly all normal.

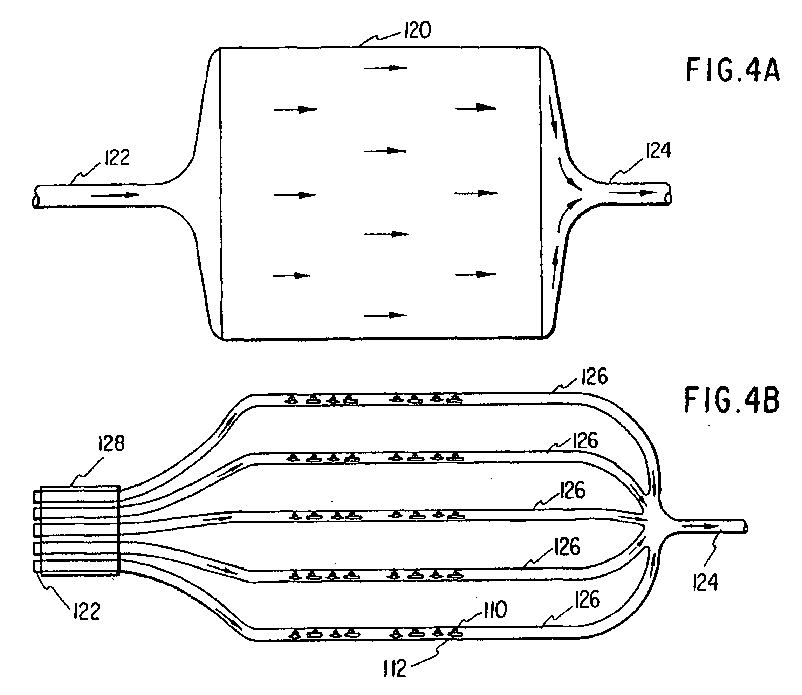

Based upon the results of these measurements, a series of parallel, rectangular chambers is established in which the rate of fluid flow (see Figs. 4a and 4b over the lower surface creates a shear stress rate which was found in the tapered chamber to remove leukemic cells from the stroma without removing all of the normal cells. In the case of chronic myelogenous leukemia patient bone marrows, this shear stress is typically 0.01-0.05 dynes/square cm. The actual flow rate employed will depend on the size and geometry of the chambers. Bone marrow cells from the patient will be cultured in these rectangular chambers at a concentration of 5 x $10^6$/ml to 50 x $10^6$/ml in Iscove's Modified Dulbecco's Medium with 5-20% (typically 10%) Fetal calf serum plus 0-15% (typically 10%) horse serum, with or without $10^6$M hydrocortisone. The bone marrow cells will be cultured for 12-24 hours without fluid flow, and then fluid flow will be initiated. The cells will be cultured for 3-7 days, at which time all of the nonadherent cells will be discarded. The adherent cells will be recovered from the rectangular plates by aspiration and mechanical agitation, and then collected. These cells can then be either directly returned to the patient, or stored in liquid nitrogen by standard techniques for later use.

Cells other than those of the hematopoietic system also may be separated using differential tolerance to shear stress. Thus, where there are distinct subpopulations of cells within a complex mixture of cells the methods described above can be used to separate out a cell type of interest from within a suspension of cells derived from, e.g. skin, liver, muscle, nerve, or epithelium. Of particular interest is the separation of tumor cells from within a population of normal cells. The population of cells to be separated will be contacted with a suitable stromal substrate as described below, such as a purified protein or cellular component to which the cells of interest adhere. The shear stress tolerance for each of the adherent subpopulations is determined as described above. The fluid flow can then be adjusted appropriately so as to retain the desired subpopulation of cells on the stroma. The desired cells are then collected as described above.

A variety of packings may be used in the reactor to provide for adherent growth of the cells, while maintaining some physical separation between the stromal cells and the hematopoietic cells, and while allowing for some contact or close juxtaposition between the stromal cells and the hematopoietic cells. In this way, the factors secreted by the stromal cells may be readily taken up by the hematopoietic cells to encourage their proliferation and, as appropriate, differentiation and maturation.

The protein matrix to support the cells may take the form of shredded collagen particles, e.g., sponges or porous collagen beads, sponges or beads composed of extra-cellular bone matrix protein from bone marrow, or protein coated membranes, where the protein may be collagen, fibronectin, hemonectin, RGDS peptide, mixed bone marrow matrix protein, or the like. Pore sizes of membranes will generally range from about 1 to 5μ to allow for interaction between the different cell types, while still retaining physical separation.

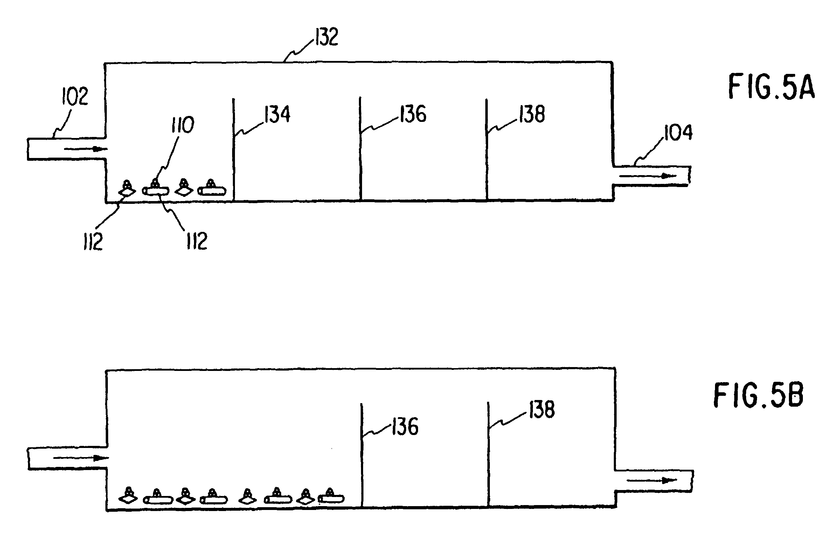

Membranes may be employed, which will be protein coated. Various membrane materials may be employed such as polypropylene, polyethylene, polycarbonate, polysulfonate, etc. Various proteins may be employed, particularly collagen or the other proteins which were indicated previously. The membrane should have sufficiently small pores, that the transformed cells may not pass through the membranes, but may grow and form a confluent layer on one side of the membrane and extend portions of the cell membrane into the pores. Generally the pores will be in the range of about 1 to 5μ. In this manner, the hematopoietic stem cells may grow on the opposite side of the membrane and interact with the transformed cells, whereby factors may be transferred directly from the transformed cells to the hematopoietic progenitor cells. The progenitor cells, the stem cells, are able to attach to the intruded cytoplasmic projections which have passed into the pores. Hematopoietic differentiation from the stem cells occurs on one side of the membrane and differentiated progeny are unable to squeeze back through the pores, which are already largely occupied by the stromal cell layer when confluence is approached or reached. For example, one could provide for a plurality of chambers in which stromal cells may grow and the hematopoietic cells may be moved in accordance with the chamber which has the stromal cells at a subconfluent level. Thus, by having a movable barrier between the chambers, when the stromal cells approach confluence, generally after about 8-12 weeks, one could open or remove the barrier between the chambers and allow for the stromal cells to migrate into the new chamber and allow for the hematopoietic cells to come in contact with the subconfluent stromal cells, while the subconfluent stromal cells feed the factors to the chamber comprising the hematopoietic cells (Fig. 5a and Fig. 5b). The transfer of the hematopoietic cells can be achieved by appropriate flow rates or by other convenient means. One can provide for various wells in the chamber, which are divided by appropriate walls, after seeding in one well, when the cells become confluent, cells will then move over into the next well and seed the next well in a subconfluent manner. Another modification of the system is one in which, after 8-12 weeks in culture, the hematopoietic cells are exposed to new, proliferating stromal cells. This is accomplished in one of several ways. This exposure to proliferating stromal cells is accomplished in one of several ways. In the first technique, the culture are exposed to EDTA for 3-5 minutes, which removes the hematopoietic stem cells from the stromal cells. The removed cells are then transferred to a new culture vessel, which may itself contain bone marrow stromal cells seeded 3-7 days prior. This process is repeated every 8-12 weeks. Another alternative approach is to add additional surface area by increasing the volume of the cultures and adding additional collagen beads to the cultures at 8-12 weeks. Finally, small organic molecules or proteins, particularly hormones, such as platelet-derived growth factor (at 100-500 ng/ml), interleukin 1 alpha, tumor necrosis factor alpha, or basic fibroblast growth factor or other molecules mitogenic to fibroblasts, can be added to the cultures every 3-7 days. This exposure to stromal mitogenic stimulatory factors promotes the continued proliferation of bone marrow stromal cells, and their continued production of hematopoietic growth factors. Thus, one can provide for the continuous subconfluent stage of the stromal cells.

Continuous fluid flow can also be used to selectively separate normal from cancerous cells within a bone marrow population. In this approach, a radial flow chamber is first used to determine the specific stromal adhesive properties of normal versus cancerous cells, and then a rectangular flow chamber with flow rates established to achieve a shear stress sufficient to remove the cancerous cells is used to preoperatively separate the normal and cancerous cells.

The subject method and apparatus also provides for the opportunity to recycle stem cells which are lost by the flow of the perfusion medium. The surface membrane protein marker CD34 substantially separates immature hematopoietic cells from mature hematopoietic cells. Thus, by capturing and recycling those cells which are CD34+, one may avoid the loss of stem cells to the medium.

Various techniques may be employed for capturing and returning the immature fraction of cells to the reactor. For example, one could label the cells with an antibody specific for CD34 and then use antibodies to the antibody for collecting the CD34+ cells and recycling them to the reactor. Alternatively to positive selection, one may use negative selection, whereby one would remove the mature cells employing antibodies to various markers associated with mature cells, such as antibodies to glycophorin A, CD33, MOI, OKT3, OKT4, OKT8, OKT11, OKT16, OKM1, OKM5 Leu7, Leu9, Leu M1, Leu M3, and the like. Various antibodies are available for markers specific for mature cells of the various hematopoietic lineages, lymphoid, myeloid and erythroid, and these antibodies may be used to remove the mature cells from the effluent from the reactor, followed by harvesting of the remaining cells and restoring them to the reactor. In this way, one can avoid forced decline in the cultures due to loss of stem cells and maintain unlimited stem survival *in vitro.*

Separation using antibody markers can be achieved in various ways, using standard techniques, individually or in combination, such as panning, fluorescence activated cell sorting, antibodies bound to various surfaces, e.g. polystyrene surface, metal microspheres and magnets, and the like. The antibodies are bound to a surface which allows for separation between adherent and non-adherent cells or the antibodies are labeled, directly or indirectly, which permits selection between labeled and unlabeled cells.

By following the subject procedures greatly extended periods of *in vitro* growth of hematopoietic cells may be achieved, generally providing *ex vivo* human hematopoiesis for at least six months in culture, with granulopoiesis being supported for at least four months and erythropoiesis for at least three months. In addition, hematopoietic progenitor cells are continuously generated throughout the culture resulting in net expansions of progenitor cells of over 10-fold from input cells.

In addition, by following the subject procedures greatly increased rates of stem cell division are supported, permitting the efficient insertion of retrovirally transfected genetic material. Genes inserted by the appropriate retroviral vector during an initial two week infection period can be expressed in up to 10-30% of all progenitor and precursor cells arising during subsequent culture for over four months in culture. These subject procedures thus support the successful transfer of genetic material into a highly proliferative human hematopoietic stem cell.

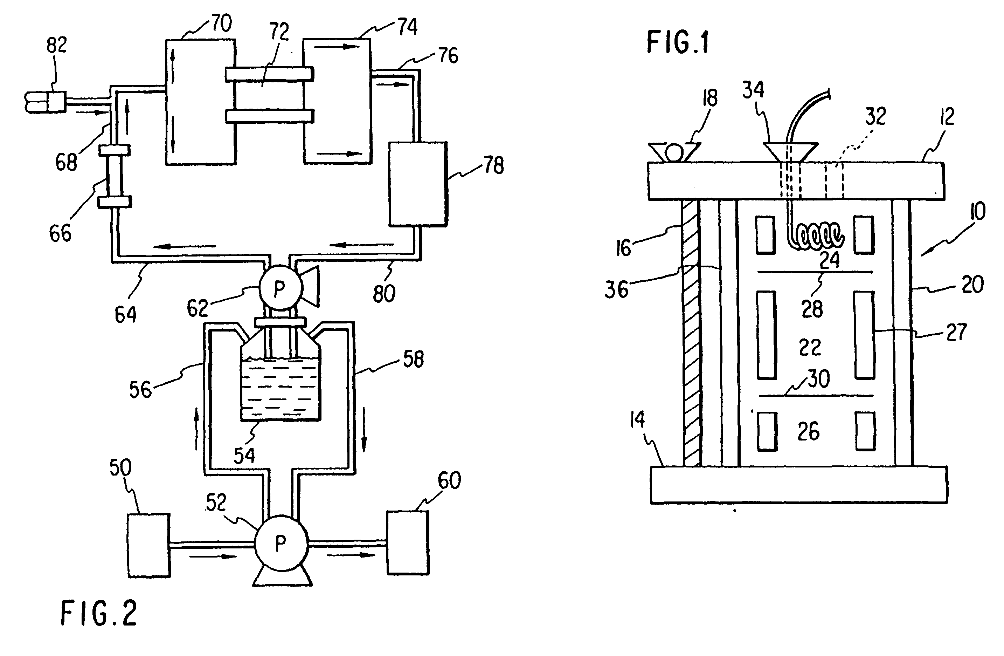

Figure 1 is a schematic view of a perfusion chamber. Reactor 10 with cover plate 12 and floor plate 14 are joined by bolts 16, held in position by wing nuts 18. Three bolts are employed, so as to avoid warping. The chamber 20 has three sections, the middle section 22 containing the support matrix for the stromal cells, the bed of stromal cells, and the bone marrow cells. The central section 22 is separated from the top section 24 and the bottom section 26 by membranes or mesh 28 and 30 respectively. Conveniently, polysulfonate membrane may be employed or a stainless steel mesh, whose mesh size is small enough so that cells are contained within the central section of the chamber. The separating interphase may be placed in the chamber using an inner cylinder 27 which is sectioned to provide the separating membrane mechanical support. The top section 24 and the bottom section 26 need not be identical and will have tubing or membranes across which liquid media and gases are exchanged. The gases are exchanged across a hydrophobic, e.g., silicone, tube whose length (and thereby gas/liquid contact area) may be varied to allow for sufficient gas fluxes to support the needs of the cell population that is metabolizing in the central section. The media can be pumped or withdrawn directly from the top or bottom sections through port 32 and may be fed through delivery tube 34.

If desired, the top and bottom sections may be eliminated by using an external oxygenator. In this situation, the separating membrane is held in place under the glass cylinder 36 which fits into cylindrical groove plates 12 and 14 and the area inside of the cylindrical groove is indented to allow for good flow distribution across the membrane. This geometry allows the fluid from the finite number of inlet ports to mix and for radial pressure to equilibrate, leading to a uniform liquid flow across the separating membrane. This setup is suitable for chambers which have relatively few cells, so that oxygenation does not become limiting.

In Figure 2 is depicted a schematic representation of the loop that connects the perfusion chamber to the side media reservoir, oxygenator, sensor chamber, and sample/injection ports.

An external fresh media source 50 is pumped by means of pump 52 to a media reservoir through line 56 and spent media is withdrawn through line 58 from reservoir 54 by means of pump 52 to the spent media container 60 for further processing. A second pump 62 pumps media from the media reservoir 52 through line 64 through a hollow fiber oxygenator 66. The media is directed through line 68 to the first chamber of bioreactor 70. As appropriate, a means for injection of media component 82 is provided, for introducing the component into line 68 for transport by the media into the first chamber of bioreactor 70. The component may be test components, additional factors, or the like. The media from bioreactor 70 is directed through central chamber 72 into the second chamber 74 of the bioreactor. From there the media is directed by line 76 to in-line sensors 78 for detecting the change in composition of the media.

For example, it is desirable that the glutamine:glucose ratio be in the range of about 1:5-8, depending on the cell lines used; for instance, preferably 1:8 for transfected 3T3 cells. Furthermore, ammonium concentrations will preferably be below about 2.0 mM and lactate concentrations are preferably less than about 40 mM. By monitoring the effluent from the bioreactor, the media introduced into the bioreactor may be modified, oxygen partial pressure may be changed, gas flow rate may be altered, various components may be augmented, or the rate of perfusion may be slowed or increased.

From the sensors 78, the media is directed through line 80 by means of pump 62 to the reservoir 54.

By means of the flow path described above, the media in the side reservoir is slowly exchanged using a separate pump. This organization allows for separate control of the media exchange rate (the outer pump) and the flow rate through the oxygenator and perfusion chamber. The former is used to control the longer term change in the media composition and perfusion, while the latter may be used to control the dissolved oxygen tension and flow patterns in the chamber. The use of a small mesh biocompatible membrane allows for plug (piston) flow in the chamber and thus allows the precise control of delivery of growth factors and other special compounds that one may wish to introduce to the hematopoietic cells and stromal cells in very precise amounts.

After autoclaving the chamber and components of the loop, the reactor is assembled in a sterile environment. The media may be circulated through the side loop and chamber for a few days while signs of contamination are monitored. If sterile assembly is accomplished, the central section of the chamber is inoculated with either the extra-cellular matrix alone or a pre-inoculated extra-cellular matrix support that contains the stromal cells. The stromal cells are then either: 1) kept in the chamber for a period of a few days while their metabolic performance and/or growth factor responsiveness is monitored and if results are satisfactory, the bone marrow is inoculated; or 2) immediately seeded with bone marrow. In either case, the cell layer is kept at the bottom of the central section of the perfusion chamber. The cells lay down additional extra-cellular matrix and the cell layer adheres to the separating membrane. At this time, the chamber may be inverted and the cell layer may then be located at the ceiling of the central section. In this configuration, the maturing cells will settle on the bottom of the central chamber as they lose their adherence to the stromal layer. This feature is important to prevent the damage caused by mature cells to the stromal layer and/ or the less mature hematopoietic cells. This feature also makes the continuous removal of mature cells easier.

These cells are harvested by withdrawing the cells by syringe, or by continuously allowing the cells to flow out of the chamber, by the pressure of the perfused medium, through the exit tubing.

The stromal cells will, for the most part, be fibroblasts transformed with one or more genes providing for desired hematopoietic growth factors. The same or different cells may be transfected with the genes, depending upon the particular selection of host cells, the same or different cells may be used for a plurality of genes.

A wide variety of normal cells or stable lines may be employed. However, it is found that not all cell strains are permissible, since transformation of some cell lines may result in the overgrowth of the cells. Desirably, the cells which are employed will not be neoplastic, but rather require adherence to a support. The mammalian cells need not be human, nor even primate. A variety of nontransformed cells may be included in the adherent cell layer as well, including normal human bone marrow adherent cells, normal human spleen adherent cells, and normal human thymic epithelium.

Methods for transforming mammalian cells, including fibroblasts, are well known and there is an extensive literature of which only a few references have been previously given. The constructs may employ the naturally occurring transcriptional initiation regulatory region, comprising the promoter and, as appropriate the enhancer, or a different transcriptional initiation region may be involved, which may be inducible or constitutive.

A large number of transcriptional initiation regions are available which are inducible or constitutive, may be associated with a naturally occurring enhancer, or an enhancer may be provided, may be induced only in a particular cell type, or may be functional in a plurality or all cell types. The transcriptional initiation region may be derived from a virus, a naturally occurring gene, may be synthesized, or combinations thereof.

Promoters which are available and have found use include the chromosomal promoters, such as the mouse or human metallothionein-I or II promoters, actin promoter, etc., or viral promoters, such as SV40 early gene promoters, CMV promoter, adenovirus promoters, promoters associated with LTRs of retroviruses, etc. These promoters are available and may be readily inserted into appropriate vectors which comprise polylinkers for insertion of the transcriptional initiation region as well as the gene of interest. In other instances, expression vectors are available which provide for a polylinker between a transcriptional initiation region and a transcriptional termination region, also providing for the various signals associated with the processing of the messenger for translation, i.e., the cap site and the polyadenylation signal. The construction of the expression cassette comprising the regulatory regions and the structural gene may employ one or more of restriction enzymes, adaptors, polylinkers, *in vitro* mutagenesis, primer repair, resection, or the like.

The expression cassette will usually be part of a vector which will include a marker and one or more replication systems. The marker will allow for detection and/or selection of cells into which the expression cassette and marker have been introduced. Various markers may be employed, particularly markers which provide for resistance to a toxin, particularly an antibiotic. Preferably, gentamicin resistance is employed, which provides resistance to G418 for a mammalian cell host. The replication systems may comprise a prokaryotic replication system, which will allow for cloning during the various stages of bringing together the individual components of the expression cassette. The other replication system may be used for maintenance of an episomal element in the host cell, although for the most part the replication system will be selected so as to allow for integration of the expression cassette into a chromosome of the host.

The introduction of the expression cassette into the host may employ any of the commonly employed techniques, including transformation with calcium precipitated DNA, transfection, infection, electroporation, ballistic particles, or the like. Once the host cells have been transformed, they may be amplified in an appropriate nutrient medium having a selective agent, to select for those cells which comprise the marker. Surviving cells may then be amplified and used.

Host cells which may be employed include African green monkey cell line CV1, mouse cells NIH-3T3, normal human bone marrow fibroblasts, human spleen fibroblasts, normal mouse bone marrow fibroblasts, and normal mouse spleen fibroblasts. It should be noted that in some instances, depending upon the choice of vector and cell line, the cells may become neoplastic. It is important that the resulting transformed cells be capable of adherence, whereby the transformed cells maintain binding to a support, such as protein sponges, protein coated membranes, or the like.

Once the vector for expressing the appropriate growth factors has been constructed, it may be used to transform the cells by any convenient means. The resulting transformed cells may then be used to seed the supports, which have already been described. These supports may be introduced into the reactor or may be present at the time of seeding in the reactor. The cells will be allowed to grow for sufficient time to ensure that the cells are viable and are capable of producing the desired growth factors.

The reactor may then be seeded as appropriate with the hematopoietic cells. The hematopoietic cells may include substantially pure stem cells, a mixture of hematopoietic cells substantially free of mature hematopoietic cells of one or more lineages, or a mixture comprising all or substantially all of the various lineages of the hematopoietic system, at various stages of their maturation .

The cells are allowed to grow with substantially continuous perfusion through the reactor and monitoring of the various nutrients and factors involved. For the most part, the primary factors will be provided by the stromal cells, so that a steady state concentration of growth factors will normally be achieved. Since conditioned supernatants are found to be effective in the growth of the hematopoietic cells, one can provide for a ratio of stromal cells to hematopoietic cells which will maintain the growth factor at a appropriate concentration level in the reactor.

Transfected stroma can provide for the introduction of genes into human stem cells. In mice, retroviral mediated gene transfer into stem cells is made possible by pretreating mice with 5-FU and then growing the harvested bone marrow cells in WEHI conditioned media, which contains IL-3 and GM-CSF (Lemischka Cell (1986) 45:917). The artificial stroma, grown with a retroviral packaging cell line secreting a retroviral vector of interest, may be used to efficiently introduce genes into human stem cells. For example, human T-cells could be made resistant to HIV infection by infecting stem cells with the retroviral vector containing an HIV antisense sequence under control of a CDC2 regulatory sequence (Greaves, Cell (1989) 56:979-986) which would allow for tissue specific expression in T-cells. There would be a factor provided by the retroviral packaging cell line essential for replication of the retrovirus; this factor would be absent in the hematopoietic target cells. Once the virus was transferred to the hematopoietic target cells, it would no able to replicate.

In Figs. 3a and b are depicted radial flow chamber 100 having inlet 102 and outlet 104, and with 25 chamber 106 where the arrows 108 indicate the direction of flow. Hematopoietic cells 110 are seeded onto a stromal layer 112 in the chamber and grown. The flow rate will determine which cells are able to adhere, the non-adherent cells 114 passing out through outlet 104.

In Figs. 4a and 4b, growth chamber 120 is provided having inlet 122 and outlet 124. In Figure 4b, inlet 122 comprises a manifold 128 which feeds individual chambers 126 containing cells 110 and stroma 112 in the chamber 126 for growth and separation.

In Figures 5a and 5b are shown growth chambers in which barriers 134, 136, 138 are removed schematically during culture: barriers 134 at about week 8-10; barrier 136 at about week 18-20 and barrier 138, at about week 28-32.

Having generally described this invention, a further understanding can be obtained by reference to certain specific examples which are provided herein for purposes of illustration only and are not intended to be limiting unless otherwise specified.

ILLUSTRATIVE CELL SEPARATION AND STAINING PROCEDURES

Separation of Bone Marrow Cells on Ficoll:

1. Dilute the bone marrow sample at a 1:4 ratio in I-MDM kept at room temperature (Iscove's Modified Dulbecco Medium; GIBCO; Cat. No. 430-2200).

2. Carefully, layer 35 ml of the diluted bone marrow sample onto 15 ml of Ficoll-Paque at room temp. (Sp. Gr. 1.077 g/cc; Pharmacia; Cat. No. 17-0840-02) in 50 ml centrifuge tube.

3. Centrifuge at 700 x g (1800 rpm on Beckman) for 30 min. at room temperature (20°C).

4. After centrifugation remove most of the upper layer (leaving about 5 ml above interphase), collect the interphase layer (bone marrow cells), and wash 3 times in ice cold I-MDM according to the following:

First wash: 1400 rpm/15 min/4°C.
Second wash: 1200 rpm/10 min/4°C.
Third wash: 1200 rpm/10 rpm/4°C.

5. After the third wash the cells are suspended either in media or a balanced salt solution etc... (depending on whtat is to be done with them), and counted after making a 1:10 dilution of the cells in acetic acid (10 µl cell suspension = 90 µl of 2% acetic acid in PBS; this will allow for counting WBCs only, since RBCs get lysed in the acetic acid).

6. Cells are then suspended to the desired final concentration in the appropriate medium (for media refer to the different applications).

Fluorescent Staining for MY-10 positive Bone Marrow Cells:

Reagents

| Standard Buffer: | |
|---|---|
| Powdered Bacto Dried DIFCO Buffer (Baxter; Cat. No. 2314-15GB): | 200 g |

# EP 0 575 350 B1

(continued)

| Standard Buffer: | |
|---|---|
| 10% NaN$_3$ (sodium azide): | 20 g |
| Heat inactivated fetal calf serum (56°C, 30 min.) | 200 ml |
| Bring volume to 20 liters in dd-H$_2$O; pH 7.15-7.25; store at 4°C | |
| Good for 1 month | |

| 2% Paraformaldehyde solution | |
|---|---|
| paraformaldehyde: | 10 g |
| dd-H$_2$O: | 500 ml |
| 10N NaOH (under the hood) | 8-20 drops |
| powdered Bacto dried Difco Buffer | 5g |
| Pour dd-H$_2$O in 500 ml flask and stir on a hot plate to 60°C in the hood. | |
| Add 10 gr. parafromaldehyde | |
| Add NaOH dropwise until solution clears | |
| Add 5 gr DIFCO | |
| Let cool, Adjust pH to 7.35-7.45 with 2 N HCl. | |

1. After counting the cells, they are washed once in standard buffer (100 rpm, 5 min. 4°C).

2. Cells are then suspended in standard buffer at the concentration of 2 x 10$^5$ cells/ml.

3. Two 50 μl aliquots of the cells are deposited into 2 15 ml centrifuge tubes.

4. To one tube 50 μl of a 1:5 dilution of anti-HPCA-1 are added (anti-human progenitor cell antigen; Becton Dickinson; Cat. No. 7660; diluted 1:5 in standard buffer). To the other tube, 50 μl of a 1:5 dilution of MIg are added (Mouse IgG1 control; Becton Dickinson; Cat. No. 9040; diluted 1:5 in standard buffer).

5. Both tubes are then incubated for 1/2 hour on ice.

6. After incubation; cells are washed twice in 5 ml standard buffer (1000 rpm, 5 min, 4°C).

7. After the second wash, cell pellets are resuspended in 50 μl of a 1:40 dilution of GAM-FITC (Affinity isolated Goat F(ab')2 anti-mouse IgG and IgM, human Ig adsorbed, Fluorescein conjugated; TAGO; Cat. No. 4353; diluted 1:40 in standard buffer).

8. Cells are incubated for 1/2 and hour in the dark, on ice.

9. After incubation, cells are washed twice in 5 ml standard buffer (100 rpm, 5 min., 4°C), and each of the pellets is resuspended in 100 μ standard buffer plus 100 μl 2% paraformaldehyde solution.

10. Cells are then analyzed for fluorescence using the flow cytometer. Percent positive fluorescence constitute % fluorescence in anti-HPCA-1 sample minus % fluorescence in MIg sample.

Fluorescent Staining of Bone Marrow Cells to Sort Out Mature Progenitor Cells:

Objectives:

The purpose of this staining is to enrich for hematopoietic stem cells (most primitve stem cells) by removing mature cell populations, using the flow cytometer or magnetic beads. It is always a good idea to retain some cells as total bone marrow cells (after Ficoll separation) to stain for MY-10 positive cells in order to compare with the outcome of sorting and determine extent of enrichment.

1. Cells are separated on Ficoll-Paque as described before. (Remove 0.5 x $10^6$ cells and divide into 2 portions to stain with anti-HPCA-1/GAM-FITC and MIg/GAM-FITC).

2. After the third wash the cell pellet is suspended in the monoclonal antibody cocktail (refer to section describing the making of this cocktail) using 1 ml of the cocktail per $10^7$ cells, and cells are incubated on ice for 1 hour.

3. Cells are then washed three times in excess ice cold I-MDM (1000 rpm, 5 min, 4°C).

4. After the third wash cells are suspended in a 1:40 dilution of GAM-FITC (diluted in I-MDM, not standard buffer) at the rate of 50 µl per 0.25 x $10^6$ cells, and incubated on ice in the dark for 1/2 an hour.

5. After incubation, cells are washed three times in ice cold I-MDM, and after the final wash they are suspended in 2-4 ml of ice cold I-MDM and kept on ice until sorting.

6. Cells are then sorted on the flow cytometer based on fluorescence, to eclude the upper 85% of the fluorescence histogram. Sorting could be repeated twice for better enrichment.

7. After sorting, cells are counted, washed, and an aliquot is stained for MY-10 positive cells (as described above) to determine the extent of enrichment in comparison to stained aliquots of the total bone marrow cells.

Selecting for Immature Cells by Using Magnetic Antibodies:

1. Follow steps 1-3 in the procedure for Fluorescent staining of bone marrow cells to sort out mature cells. Note: sodium azide is not included in any of the buffers.

2. In the meantime wash an appropriate amount of magnetic goat anti-mouse Ig (Biomag; Collaborative Research; Cat. No. 74340-50; 1 mg/ml; 5 x $10^8$ particles/ml) 3 times in ice cold I-MDM at 1500 rpm, 5 min. 4°C (to wash off the sodium azide which is used as a preservative).

3. Resuspend the cell pellet obtained after the third wash in "step 1" in Biomag at the rate of 50 particles Biomag/ cell (e.g., for 1 x $10^6$ cells use 5 x $10^7$ particles, therefore, 0.1 ml of Biomag).

4. Deposit the cells in a T-25 or T-75 tissue culture flask (depending on cell numbers) and incubate on ice for 1/2 hour with intermittent shaking.

5. After incubation, lay the flask onto the flat magnet (provided with the Biomag), secure with a rubber band or tape, and incubate at 4°C for 10-15 min.

6. Stand the magnet and the flask into an upright position and collect the supernatent.

7. Repeat steps 4-6 two more times.

8. Count the cells, wash once in ice cold I-MDM, remove an aliquot to stain for MY-10 positive, and resuspend in appropriate media for further use.

I. Medium Replacement

Materials and Methods:

Cells: Human bone marrow cells were obtained from heparinized aspirates from the iliac crest of informed and consenting individuals. The bone marrow was separated by a Ficoll-Paque (Pharmacia, No. 17-0840-02) density gradient centrifugation and the low density cells (<1.077 gm/cm$^3$) were collected and washed 3 times with Iscove's Modified Dulbecco's Medium (IMDM). The cells were counted between the second and third washes. The cells were then seeded onto 24-well tissue culture plates (Costar No. 3524) in duplicate or triplicate at 1, 2, and 5·$10^6$ cells/ml at

322 µl/well.

<u>Long-term culture conditions</u>: The low density cells were incubated in IMDM supplemented with 10% fetal calf serum (Hyclone Laboratories, 10% horse serum (Hyclone Laboratories), 1% penicillin/streptomycin (Sigma, 10,000 U/ml penicillin G and 10 mg/ml streptomycin, Cat. No. P3539), and $10^{-5}$ M hydrocortisone (17-Hydroxycorticosterone, Sigma, Cat. No. H0888) in a humidified 5% $CO_2$/95% air atmosphere. The cultures were treated with one of three medium exchange schedules, 100% daily medium exchange (7/wk), 50% daily medium exchange (3.5/wk), or 50% biweekly medium exchange (1/wk). Twice per week during the medium exchange, 50% of the non-adherent cells were removed from each culture well and counted using a hemocytometer.

When the cells were removed for counting (twice/week), all of the medium removed during feeding of the 3.5/wk and 1/wk cultures was saved for cell counts and fresh medium returned to the wells. The 7/wk cultures required saving 1/2 of the removed medium for cell counts, while centrifuging and returning the non-adherent cells in the remaining 1/2 of the medium removed. Fresh medium was then added to each well to replace the medium removed for cell counts. On days when the cells were not removed for counting, 100% or 50% of the medium was removed from each of the 7/wk and 3.5/wk culture wells respectively, the cells were centrifuged and returned to the original wells with additional fresh medium.

<u>Methylcellulose and morphologic assays</u>: One every other week the non-adherent cells removed for cell counts were plated in methylcellulose in the presence of erythropoietin, GM-CSF, and IL-3, and the Granulocyte Macrophase-Colony Forming Units (CFU-GM) were enumerated. Aliquot of removed cells were cytocentrifuged, stained with Wright-Giemsa, and differential cell counts performed.

<u>Statistical analysis</u>: The biweekly cell production results are expressed as the mean ±SEM from replicate cultures. The probability of significant differences between groups of cultures was determined by comparing the normalized cumulative cell production values from the rapidly exchanged cultures (7/wk and 3.5/wk) to the matched control cultures (1/wk) using a paired t-test. Statistical significance was taken at the 5% level.

Results:

<u>Kinetics of nonadherent cell production</u>: Nonadherent cell production was examined both as a function of inoculum cell density (over the range 1-5-$10^6$ cells/ml) and medium exchange rate. The medium exchange rate was varied from one medium volume exchange per week, the traditional Dexter culture rate, to seven medium volume exchanges per week. The biweekly number of cells collected was normalized by dividing by the number of cell inoculated per culture.

At each medium exchange rate, the normalized cell collection curves did not change significantly with inoculum density. The cell production for the cultures maintained at the three medium perfusion rates of 7/wk, 3.5/wk and 1/wk were similar when normalized to the number of cells inoculated per culture. Comparison of the final cumulative cell productions between inoculum densities showed no significant differences, at any of the three medium exchange rates (p > .20 by a paired t-test for all pairs of samples).

The medium exchange rate, in contrast, strongly influenced the rate and longevity of cell production in these cultures. Cell production of the cultures exchanged at 1/wk (control), 3.5/wk, and 7/wk all decayed over the first few weeks. Differences in culture productivity, however, became apparent after week 3 in culture. Between weeks 3 to 10, the cell production was constant in the 7/wk cultures, constant at a lower level in the 1/wk cultures, but increased exponentially in the 3.5/wk cultures. After weeks 10 to 12, cell production declined in all cultures until culture termination.

Results for the 1/wk exchanged cultures are equivalent to those commonly observed in traditional human Dexter cultures in a variety of systems, whereas the rapidly exchanged cultures of 3.5 and 7/wk showed increased cell productivity when compared to previous optimum culture methods. Cultures in which 1/2 of the medium was exchanged daily (3.5/wk) maintained increased cell production for substantially longed than either the control (1/wk) or complete daily exchange (7/wk) cultures. Between weeks 3 and 9, the number of nonadherent cells collected from the 3.5/wk exchanged cultures increased exponentially with a doubling every 2.1 weeks.

The cell production under the 3.5/wk and 1/wk protocols can be directly compared by plotting the cell production under the 3.5/wk exchange rate as a percentage of the production of the cultures with an exchange rate of l/wk. This comparison shows that during the initial decay phase the cell production under the two protocols is similar. However, between weeks 3.5 and 18, the cell production under the 3.5/wk exchange rate is consistently higher.

The proliferative potential of the cultures can thus be measured by their ability to produce cells following the initial decay. The normalized cumulative cell production following week 3 ($\Sigma_{i=7}^{n}$, $C_i/C_o$ was independent of the cell inoculation density for the medium exchange rates of 7/wk, 3.5/wk. Cell production data from the cultures at similar medium exchange rates were qualitatively and statistically similar, and were therefore density averaged and combined (bottom panel) to obtain a larger statistical sample. The density averaged cumulative cell production between weeks 3.5 and 20 was: 0.22 for the 7/wk; 0.40 for the 3.5/wk; and 0.15 for the 1/wk cultures. The increase in the medium exchange rate from 1/wk to 7/wk thus increased the cell production about 60% over the typical Dexter culture medium exchange schedule. The 3.5/wk exchange rate resulted in almost 3-fold cumulative cell production increase compared to the 1/wk Dexter protocol. Statistical analysis of these data using a paired t-test, demonstrated significant differences between both the 7/wk vs. 1/wk and the 3.5/wk vs. 1/wk at the 5% level of significance. The medium exchange rate of 3.5/wk thus improves the cell production rate over the traditional Dexter protocol of 1/wk.

Granulocyte-macrophase progenitor cell production:

Granulocyte-macrophase progenitor cell assays were performed from replicates of a given medium perfusion schedule and inoculum density (Table 2). The medium perfusion rate had a pronounced effect on the number of granulocyte-macrophage progenitor cells produced. The 3.5/wk medium exchange cultures showed the greatest longevity in terms of progenitor cell production. These cultures produced progenitors at a stable rate between weeks 4 and 18.

The optimum conditions in terms of progenitor cell production are the cultures exchanged 3.5 times per week and inoculated at $5 \cdot 10^6$ cells/ml. These cultures produced a significant number of progenitor cells until week 20. Statistical analysis, using a paired t-test, showed that the optimum medium exchange rate cultures of 3.5/wk produced significantly more granulocyte-macrophage progenitor cells after week 8 than did the corresponding 7/wk and 1/wk cultures at all three inoculation densities at the 1% level of significance. The number of progenitor cells produced is important as it is an indirect measure of stem cell renewal. Progenitor cells can only be present after several weeks in culture by differentiation from an earlier cell, presumably a stem cell, which is still present in culture. Thus, these data suggest that more physiologic, rapid medium/serum exchange rate and higher cell densities may have provided conditions that supported some degree of stem cell renewal for five months.

Nonadherent cell morphology: To determine whether the prolonged hematopoiesis supported by the 3.5/wk cultures was qualitatively different from the other cultures, the non-adherent cells collected between weeks 10 and 19 were stained and typed morphologically. At the exchange rates of 1/wk and 7/wk, the cells produced were mostly macrophages by week 15 and thereafter (Table 3), which is similar to results from studies in other laboratories. In contrast, the cultures perfused at a rate of 3.5 medium volumes per week and seeded at $5 \cdot 10^6$ cells/ml produced granulocytes as well as macrophages through week 19. Thus, it seems that this medium exchange rate and inoculum density more effectively reconstituted granulopoiesis *in vitro*.

Table 2:

| The average number of nonadherent progenitor cells removed from long term bone marrow cultures (LTBMCs) as a function of the medium perfusion rate and inoculum density. | | | | | | | | | |
|---|---|---|---|---|---|---|---|---|---|
| Week | $5 \times 10^6$ per ml | 7/wk $2 \times 10^6$ per ml | $1 \times 10^6$ per ml | $5 \times 10^6$ per ml | 3.5/wk $2 \times 10^6$ per ml | $1 \times 10^6$ per ml | $5 \times 10^6$ per ml | 1/wk $2 \times 10^6$ per ml | $1 \times 10^6$ per ml |
| 2 | 237±27 | 11±3.3 | 106±5 | 120±16 | 132±7.9 | 167±13 | 368±29 | 94±20.8 | 335±46 |
| 4 | 149±21 | 101±5.1 | 104±10 | 93±10 | 37±5.6 | 20±0 | 21±1.3 | 2±0 | 8±4.4 |
| 6 | 47.7±7 | 12±2.5 | 8±0 | 17±3 | 6±4.1 | 5±2.7 | 13±5.1 | 1±0 | 1±0 |
| 8 | 40±3 | 0 | 4±0 | 38±6 | 24±2.7 | 10±3 | 34±7.4 | 0 | 0 |
| 10 | 0 | 0 | 0 | 28±8.3 | 10±2.9 | 5±1.3 | 8±2.3 | 2±2.3 | 0 |
| 12.5 | 0 | 6±2.3 | 0 | 8±2.3 | 0 | 0 | 0 | 0 | 0 |
| 14 | 0 | 0 | 0 | 22±6.4 | 6±1.3 | 2.5±1.2 | 3±1.3 | 0 | 0 |
| 16 | 6±2.2 | 0 | 0 | 24±7.6 | 4±1.7 | 2±1.3 | 9±3.6 | 0 | 0 |
| 18 | 0 | 0 | 0 | 24±6.3 | 4±1.3 | 0 | 0 | 0 | 0 |
| 20 | 0 | 0 | 0 | 5±0 | 4±0 | 3±0 | 1±0 | 0 | 0 |
| 22 | 2±1.3 | 0 | 0 | 4±1.3 | 10±3 | 0 | 0 | 0 | 0 |
| 10-22* | 8±3.5 | 6±2.3 | 0 | 115±32.2 | 40±11.2 | 12.5±3.8 | 21±7.2 | 2±7 | 0 |
| Replicate samples at each medium perfusion rate and inoculum density were pooled and are each tabulated as one mean ± SEM. Cumulative CFU-GM production after week 8 is statistically greater in the 3.5/wk cultures than the corresponding cultures perfused at 7/wk or 1/wk at all inoculum densities at the 1% level of significance. | | | | | | | | | |

This result supports the hypothesis that long-term human Dexter culture conditions are suboptimal and as a culture *in vitro* better approximate the hematopoietic environment *in vivo*, more effective reconstitution of bone marrow *ex vivo* can be attained.

Physical appearance: The medium exchange rate significantly affected the physical appearance of the cultures. By 10 weeks in culture, the 7/wk cultures had large number of adipose cells in the stroma while the 3.5/wk cultures had few fat cells and the 1/wk cultures never developed fat cells. At culture termination at 26 weeks, the stroma of the 7/wk cultures were composed of approximately 20-30% fat cells while the 3.5/wk cultures still only had a few fat cells. Adherent colony distribution also varied between cultures with different medium perfusion rate. Adherent colonies in the 3.5/wk cultures persisted longer than those in the 7/wk and 1/wk cultures.

**Table 3: Nonadherent cell morphology as a function of the medium perfusion rate and inoculum density.**

| | | $5 \times 10^6$ per ml | | | $2 \times 10^6$ per ml | | | $1 \times 10^6$ per ml | | |
|---|---|---|---|---|---|---|---|---|---|---|
| Medium perfusion rate | weeks | %Mø | %G | % myeloid pre-cursors | %Mø | %G | % myeloid pre-cursors | %Mø | %G | % myeloid pre-cursors |
| 7/wk | 10.4 | 25 | 57 | 18 | 57 | 32 | 11 | 52 | 34 | 14 |
| | 13.4 | 49 | 34 | 17 | 92 | 5 | 3 | 63 | 22 | 15 |
| | 15.4 | 66 | 19 | 16 | 79 | 19 | 2 | 54 | 17 | 29 |
| | 19 | 93 | 5 | 1 | 96 | 3 | 1 | 100 | 0 | 0 |
| 3.5/wk | 10.4 | 50 | 27 | 23 | 45 | 38 | 17 | 39 | 45 | 17 |
| | 13.4 | 23 | 59 | 19 | 27 | 56 | 17 | 36 | 47 | 17 |
| | 15.4 | 41 | 38 | 21 | 44 | 27 | 29 | 67 | 13 | 21 |
| | 19 | 58 | 37 | 5 | 88 | 9 | 3 | 99 | 1 | 0 |
| 1/wk | 10.4 | 59 | 21 | 20 | 60 | 11 | 29 | ND | ND | ND |
| | 13.4 | 56 | 25 | 20 | 19 | 36 | 46 | 43 | 7 | 50 |
| | 15.4 | 76 | 4 | 20 | ND | ND | ND | 46 | 39 | 15 |
| | 19 | 100 | 0 | 0 | 100 | 0 | 0 | 100 | 0 | 0 |

Data are for pooled replicate samples at each medium perfusion rate and inoculum density and are shown as the percentage of macrophages (%Mø), granulocytes (mature granulocytes and bands, %G), and immature granulocytes (metamyelocytes and less mature cells, % myeloid precursors).

II. Medium Replacement Combined with Supplementation of Medium with Hematopoietic Growth Factors

Materials and Methods:

Cells: Human bone marrow cells were obtained following informed consent from heparinized aspirates of the iliac crest bone marrow, under a protocol approved by the University of Michigan Human Investigation Committee. The bone marrow was separated by a Ficoll-Paque (Pharmacia) density gradient centrifugation and the low density cells (<1.077gm/cm$^3$) were collected and washed 3 times with IMDM. The cells were counted between the second and third washes. The cells were then seeded onto 6-well tissue culture plates (Costar No. 3406) or collagen coated 6-well plates (rat tail type 1 collagen, Biocoat. Collaborative Research Inc. Cat. No. 40400) in duplicate $5 \cdot 10^6$ cells/ml at 1.5 ml/well.

<u>Culture medium</u>: The medium used was IMDM (Gibco Laboratories. Cat. No. 430-2200) containing 10% fetal calf serum (Hyclone Laboratories), 10% horse serum (Hyclone Laboratories), 1% penicillin/streptomycin (Sigma, 10,000 U/ml penicillin G and 10 mg/ml streptomycin, Cat. No. P3539), and $10^{-5}$M hydrocortisone (17-Hydroxycorticosterone, Sigma, Cat. No. H0888).

<u>Hematopoietic growth factors (HGH)</u>: Due to the frequent culture supplementation via rapid medium exchange, hematopoietic growth factors were added to the medium at approximately 1/20 of the concentrations found to promote maximal colony formation in clonal assays 4. The concentrations used were 1 ng/ml of IL-3, 1 ng/ml of GM-CSF (Amgen Biologicals, Cat. No. 13050), 0.1 U/ml of Epo (Terry Fox Labs. Vancouver, Canada).

<u>Hematopoietic progenitor cell assay</u>: Nonadherent hematopoietic cells removed from culture were counted and plated at $1 \cdot 10^5$ cells/ml or fewer cells in methylcellulose. GM-CSF and Epo were added to the methylcellulose at 20 ng/ml and 2 U/ml, respectively. The cell were plated in 24 well plates at 0.25 ml/well and incubated at 37°C for 14 days. The colonies were then counted under an inverted microscope and colonies greater than 50 cells were scored as GM-colony forming units (CFU-GM), erythroid burst-forming unit (BFU-E), or granulocyte erythroid megakaryocyte macrophage-colony forming unit (CFU-GEMM).

<u>LTBMC conditions</u>: The cultures were incubated at 37°C in a humidified 5% $CO_2$/95% air atmosphere and perfused (medium exchanged) at a rate of 50% daily medium exchange. During the first week in culture, all cells removed during the daily medium exchange were centrifuged and returned to the original wells. After the first week in culture, 50% of the total nonadherent cells were removed from the cultures on a biweekly basis during the medium exchange, mononucleated cells counted, and fresh medium returned to the wells. The remaining five days per week when the cells were not counted, 50% of the medium was removed from each of the culture wells and replaced with fresh medium, the removed medium was centrifuged, the medium decanted from the cell pellet, and the cells returned to their original wells.

<u>Statistical analysis</u>: The probability of significant differences between groups of cultures was determined by comparing the normalized cumulative cell production values from the rapidly perfused cultures supplemented with hematopoietic growth factors to the matched untreated control cultures using a paired t-test. Statistical significance was taken at the 5% level. There were no statistical differences between matched rapidly perfused LTBMCs cultured on tissue culture plastic and type I rat tail collagen at the 5% level. Therefore, the data for the plastic and collagen matrix were combined for presentation in this and all other figures and statistical analysis performed on the combined data.

Results:

<u>Kinetics of cell production in rapidly exchanged growth factor supplemented LTBMCs</u>: As a first test of the hypothesis that the longevity and productivity of long term bone marrow cultures (LTBMCs) is limited by insufficient production of HGF's, we maintained rapidly exchanged ex vivo bone marrow cultures that were supplemented with IL-3, or Epo. In these cultures, 50% of the medium was removed daily and replaced with an equal volume of fresh medium supplemented with IL-3 or Epo. The cells removed were then centrifuged, the medium decanted and discarded, the cells resuspended, and the cells returned to the original cultures. IL-3 and Epo individually enhanced the cell productivity of rapidly exchanged LTBMCs. The cultures containing Epo alone initially had a high cell production rate due to substantial terminal erythroid differentiation. However, by week four erythropoiesis had ceased and the cell production rate had decreased to the level of the control cultures. IL-3 and Epo induced an average increase in nonadherent cell production over controls throughout the 18 weeks of culture of 175% and 173%, respectively.

Combinations of growth factors proved to be more effective in increasing the nonadherent cell production rate. The highest rate of cell production was observed for the combination of IL-3+GM-CSF+Epo. These cultures produced approximately 25% of the number of cells inoculated biweekly during the first 6 weeks in culture and had an average 4.8-fold increase in nonadherent cell production over controls during weeks 2-8. The combination of IL-3+GM-CSF produced an average 3.5-fold increase in nonadherent cells as compared to controls through week 8. In separate experiments, adding neither IL-6 nor G-CSF to the combination of IL-3+GM-CSF+Epo improved the nonadherent cell production rate, but instead resulted in cell production rates indistinguishable from the cultures containing the combination of IL-3+GM-CSF. In all cases, the stimulatory effect on cell production induced by the addition of HGFs was maximal between weeks 0 to 8, although cell production was higher than the controls throughout the culture.

The combinations of HGFs lead to high absolute numbers of nonadherent cells produced in rapidly exchanged LTBMCS. The productivity of the cultures can be shown by comparing the cumulative number of cells produced over time ($\Sigma_{i=1}^{n}$, $C_i$, $C_i$ being the number of nonadherent cells collected at time $i$), relative to the number of cells inoculated ($C_o$) by plotting the ratio ($\Sigma_{i=1}^{n}$, $C_i$, $C_o$) as a function of time. When this ratio exceeds unity, a culture has produced more cells than were inoculated and the culture has led to an expansion in cell number.

The combination of IL-3+GM-CSF+Epo induced cumulative cell production that was more than 3-fold greater than the number of cells inoculated. The cell production rate was the highest during the first 6 weeks in culture during which time the culture produced approximately as many cells as were inoculated every two weeks. This maximum cell production rate was 15% of the estimated *in vivo* bone marrow cell production rate where 50% of the myeloid cell mass is generated daily. The combination of IL-3+GM-CSF resulted in more than a 2-fold expansion in cell number and at rates comparable to the combination of IL3+GM-CSF+Epo during weeks 3-7 in culture. Untreated rapidly exchanged (50% daily medium exchange) and slowly exchanged (50% medium exchange biweekly) control cultures not supplemented with HGFs produced approximately 1 and 0.37 times the number of cells inoculated after 18 weeks, respectively. More importantly more than half of all cells removed from these unsupplemented cultures came from the first two samplings, indicating that many of these cells were from the original inoculum and that supplementation of the cultures with HGFs are required to induce significant cycling of progenitor and stem cells.

Morphologic analysis of nonadherent cells: The addition of multiple HGFs also increased the variety of myeloid cells produced in the cultures. The control cultures produced nonadherent cells that were predominately macrophages after week 3 in the culture. Production of erythroid cells decreased rapidly with few erythroid cells detected after week 5. The cultures containing Epo (Epo alone, IL-3+Epo, and IL-3+GM-CSF+Epo) produced a transient increase in erythroid cell production, with a high percentage (55-75%) of nonadherent cells being erythroid through week 3. When IL-3+Epo±GM-CSF was present, the cultures continued to produce erythroid cells throughout the 16 weeks in culture with about 5-15% of the nonadherent cells being typed as erythroid. Thus, in the presence of IL-3+Epo, erythropoiesis was active throughout.

IL-3 ± Epo led to a nonadherent cell population that was predominately (60-70%) late granulocytes (LG) at week 5. The percentage of LGs steadily declined until it reached about 20% at week 18. The production of macrophages rose correspondingly. When GM-CSF was added to IL-3±Epo, the high percentage of LG persisted through 18 weeks. The combination of IL-3+GM-CSF thus led to active granulopoiesis for 18 weeks in culture, and the addition of Epo maintained erythropoiesis as well. Photomicrographs of the control and IL-3+GM-CSF+Epo supplemented cultures at 5.5 weeks in culture show the dramatic enhancement in culture density and variety of cells produced.

Kinetics of nonadherent progenitor cell production: Progenitor cell production increased with the addition of multiple HGFs. The production of granulocyte macrophage colony forming units (CFU-GMs) in the untreated controls was prolonged and steady for over 18 weeks, which is consistent with the earlier results obtained using rapidly perfused LTBMC without HGF. CFU-GM produced in the IL-3+GM-CSF and IL-3+Epo±GM-CSF cultures was approximately 10-fold higher than controls during weeks 3 to 5.

Erythroid burst forming unit (BFU-E) production in human LTBMC has been reported to be low and cease quickly (Coutinho et al, Blood (1990) 75(11): 2118-2129). The rapidly exchanged, untreated controls exhibited a rapid decrease in BFU-E production although low levels of BFU-E were produced through 17 weeks in culture. The addition of Epo alone did not significantly influence the number of BFU-Es produced. IL-3 alone induced a mild short-lived stimulation of BFU-E production in weeks 3-5. On the other hand, IL-3 plus either Epo or GM-CSF induced a 10 to 20-fold elevation of nonadherent BFU-E levels compared to that of controls during weeks 3 to 5 of culture.

## III. Transformation of human stem cells

### Materials and Methods

Cells Human bone marrow cells were obtained following informed consent from heparinized aspirates of the iliac crest bone marrow, under a protocol approved by the University of Michigan Human Investigation Committee. The bone marrow was separated by a Ficoll-Paque (Pharmacia) density gradient centrifugation and the low density cells (<1.077 gm/rm$^3$) were collected and washed 3 times with IMDM. The cells were counted between the second and third washes. For the CD18 gene transfer experiments, bone marrow was obtained following informed consent from a CD18 deficient patient donor.

Lineage negative (Lin⁻) selection of bone marrow cells Mature mononuclear cells were removed from the above cell preparation by incubating the cells with a mixture of monoclonal antibodies (MAb) after the third wash with IMDM. 10$^7$ cells were incubated in 1ml of MAb cocktail on ice for 1 hour with gentle mixing every 10-15 minutes. The MAb cocktail used is shown in Table 4.

Table 4:

| Preparation of monoclonal antibodies used to separate lineage[+] bone marrow mononuclear cells. | | | | |
|---|---|---|---|---|
| Monoclonal Antibody | Specificity | Cat. No. | Conc. µg/10$^9$ cells | Volume (µl) |
| Anti-Leu-1 | T-cell | 6300 | 30 | 125 |
| Anti-Leu-5b | T-cell, E-rosette receptor | 7590 | 32 | 16 |
| Anti-Leu-10 (Anti-HLA-DQ) | B-celle, monocytes | 7450 | 250 | 125 |

Table 4: (continued)

| Preparation of monoclonal antibodies used to separate lineage+ bone marrow mononuclear cells. | | | | |
|---|---|---|---|---|
| Monoclonal Antibody | Specificity | Cat. No. | Conc. μg/10⁹ cells | Volume (μl) |
| Anti-Leu-12 | B-cells | 7540 | 120 | 60 |
| Anti-CALLA | Common Acute Lymphoblastic Leukemia | 7500 | 60 | 60 |
| Anti-Leu-M1 | Monocytes, granulocytes | 7420 | 1000 | 500 |
| Anti-MO-1 | Macrophages | - | 1000μl | 1000 |
| Anti-10F-7 | RBCs | - | 2000μl | 2000 |
| Anti-E3 | RBCs | - | 2000μl | 2000 |
| Add IMDM to make MAb cocktail total volume of 100 ml, filter sterilize, aliquot in 1 ml columns, and store at -20°C. | | | | |

The cells were washed 3 times in excess ice cold IMDM and centrifuged at 4°C. An appropriate amount of magnetic goat anti-mouse Ig (Biomag; Collaborative Research Corp., Cat. No. 74340-50, 1mg/ml, 5x10⁸ particles/ml) was washed 3 times in ice cold IMDM and centrifuged. The cells were resuspended in Biomeg at 50 particles/cell and placed in a T-25 or T-75 tissue culture flask and incubated on ice for ½ hour with intermittent shaking. After incubation, the flask was laid onto flat magnet, the magnet secured to the flask and incubated at 4°C for 10-15 minutes. The magnet and flask were stood upright and the supernatant collected. The incubation with shading, adding magnet, standing upright, and collecting supernatant was repeated 2 more times. The cells were counted and seeded onto 6-well tissue culture plates. (Costar No. 3406).

Culture medium The medium used was IMDM (Gibco Laboratories, Cat. No. 430-2200) containing 10% fetal calf serum (Hyclone Laboratories), 10% horse serum (Hyclone Laboratories), 1% penicillin/streptomycin (Sigma, 10,000 U/ml penicillin G and 10 mg/ml streptomycin, Cat. No. P3539), and 10⁻⁵M hydrocortisone (17-Hydroxycorticosterone, Sigma, Cat. No. H0888).

Hematopoietic growth factors The hematopoietic growth factors were the optimum discussed above. The concentrations used were 1 ng/ml or 0.4 U/ml of IL-3 (a gift from Genetics Institute, Cambridge, MA), 1 ng/ml of GM-CSF (a gift from Genetics Institute, Cambridge, MA), 50 U/ml of IL-1a (Genzyme Corp.), 0.1 U/ml of Epo (Terry Fox Labs, Vancouver Canada), 10 ng/mi MGF (mast cell growth factor, the c-kit ligand, Immunex Corp., Seattle, WA), and 2.0 ng/ml Hybrikine ([PIXY321] Immunex Corp., Seattle, WA).

Hematopoietic progenitor cell assay Nonadherent hematopoietic cells removed from culture during the weekly sampling were counted and plated at 1-10⁵ cells/ml or fewer cells in methylcellulose. MGF, GM-CSF and Epo were added to the methylcellulose at 50 ng/ml, 20 ng/ml, and 2 U/ml, respectively. The cells were plated in 24 well plates at 0.25 ml/well and incubated at 37°C for 14 days. The colonies were then counted under an inverted microscope and colonies greater than 50 cells were scored as a GM-colony forming units (CFU-GM), erythroid burst-forming unit (BFU-E), or granulocyte erythroid megakaryocyte macrophage-colony forming unit (CFU-GEMM).

Retroviral producer cell lines Two retroviral producer cell lines were obtained from Dr. Eli Gilboa's Laboratory at Memorial Sloan Kettering Cancer Center, New York, NY. The cell line produce amphotrophic viral particles that contain the NEO gene which produces neomycin phosphotransferase providing resistance to the mammalian neomycin analog G418. Also both cell lines produce retroviral particles that are deficient in the required retroviral genes so that cells infected with the retrovirus cannot themselves produce infectious virus.

The SAX containing packaging cell line is a 3T3 based cell line which contains a modified Moloney Murine Leukemia Virus (MoMuLV). The SAX provirus contains the NEO gene, an SV40 promoted Adenosine deaminase gene, in a XhoI restriction site. Also, the SAX provirus contains the ψ packaging region but is deficient in the gag (core proteins), pol (reverse transcriptase), and env (envelope proteins) genes. This second retroviral particle contains a double copy of the foreign DNA and the retroviral particles are denoted DC-29 (double copy-29th clone). The DC-29 provirus contains two copies of the NEO gene and other retroviral and foreign DNA in a 3T3 cell line.

For the CD18 experiments, the amphotropic packaging cell Psi-Crip infected with a retroviral vector containing a human full length CD19 cDNA was used (Wilson et al, Science (1990) 248:1413-1416). In this retrovirus, a full length cDNA for human CD18 was cloned into the BamH1 site of a vector that expresses the recombinant gene from heterologous sequences spanning the 5' region of the chicken β-actin gene called BA-CD18. Sequences 5' to the immediate early (IE) gene of human cytomegalovirus were subcloned into PUC19 and a portion containing IE enhancer sequences was removed on a Xho 1(from the polylinker) to Nco 1(-220 of the IE gene) fragment). Synthetic linkers were used to convert the Ncol site to a Xhol site and the modified fragment was cloned into the unique Xhol site of BA-CD18 located 5' to the β-actin promoter. This new vector was called CMV-BA-CD18.

Retroviral particle production The SAX retroviral particles were provided by Dr. Clay Smith in Dr. Eli Gilboa's laboratory as viral supernatant solutions frozen and stored at -80°C. The DC-29 and CD18 retroviral particles were produced by growing the DC-29 and CD18 viral packaging line to near confluency in a T-75 flask, changing all the medium, incubating the cells for 12-15 hours and then collecting the medium containing the viral particles. The virus containing supernatant was then centrifuged to remove vital packaging cells, the medium removed, and frozen in aliquots at -80°C.

LTBMC with supernatant added SAX retrovirus. DC29 retrovirus, or CD18 retrovirus The cultures were incubated at 37°C in a humidified 5% $CO_2$/95% air atmosphere. During the two first weeks in culture, two thirds of the medium (1 ml) was removed from each culture well daily and the medium replaced with an equivalent volume fresh medium containing HGP's (0.85 ml) and viral cell producer supernatant; (0.15 ml). The retroviral supernatant containing medium was thawed immediately prior to use and if not completely urged, it was stored on ice in a refrigerator. The medium removed from the cultures was centrifuged, the medium decanted, and the cells returned to the original wells.

LTBMC co-cultured with SAX retrovirus packaging cell line The SAX retrovirus packaging cell line was grown to approximately 10% confluency in T-25 flasks (Costar, No. 3056) and then subjected to 2000 rads of radiation. The hematopoietic cells prepared above were added to the irradiated viral producer cells and cultured wit 50% daily medium exchange for 2.5 weeks with all cells being returned to the wells. At 2.5 weeks in culture, a 0.5 mM solution of EDTA was added to the flasks to remove the hematopoietic cells while leaving the stroma. The removed hematopoietic cells were added to 3 wells of a 6 well plate with 1000 freshly trypsinized bone marrow fibroblast cells per well.

Sampling of infected LTBMCs Beginning at week two in culture, after retrovirus addition had ended or co-culture had ceased, the cultures had 50% medium exchanged per day with the nonadherent cells in the exchanged medium being removed once per week for analysis. Nonadherent cells were removed from the cultures during the daily medium exchange, the mononucleated cells counted, and fresh medium returned to the wells. The remaining six days per week when the cells were not counted, 50% of the medium was removed from each of the culture well sand replaced with fresh medium, the removed was centrifuged, the medium decanted from the cell pellet, and the cells returned to their original wells.

Analysis for retroviral infection The initial bone marrow inoculum was plated in 0, 0.4, 0.8, 1.2, 1.6, and 2.0 mg/ml G418 to obtain a kill curve to determine the concentration of G418 in which to plate the post-infected bone cells. Cells removed from the cultures were plated in methylcellulose with G418 at 0.0, 0.8, and 1.6 mg/ml G418. After two weeks, the number of progenitor cell colonies were enumerated in the methylcellulose. Individual colonies were then plucked from the methylcellulose and assayed by polymerase chain reaction (PCR) for retroviral DNA.

Statistical analysis The probability of significant difference between groups of cultures was determined by comparing the cumulative cell production values from the experimental samples to the matched control cultures using a paired t-test. Statistical significant was taken at the 5% level.

Results:

Retroviral infection using SAX retrovirus

Kinetics of cell production in LTBMCs infected with SAX retrovirus Cell production in retrovirally-infected cultures is an indicator of the likelihood of retroviral infection and is therefore a useful parameter to measure. Retroviral integration into the target cell genome is only thought to occur during cell division. for this reason, increased culture productivity increases the probability of stem cell mitosis and thereby increases the probability of retroviral infection. The highest cell production occurred in the cultures with supernatant virus addition supplemented with IL-3+GM-CSF and IL-3+GM-CSF+IL-1α which produced increasing number of cells through 4 weeks in culture. The LTBMCs co-cultured with the SAX virus packaging cells produced more cells than the supernatant addition cultures at week two, although cell production decreased after week 2.

Analysis of retroviral infection in LTBMCs with supernatant SAX virus addition The percentage of progenitor cells surviving in high [G418] varied from 2 to 50% in the cultures supplemented with IL-3+GM-CSF or IL-3+GM-CSF+IL-1α during the first four to six weeks in culture. By 10 weeks in culture (8 weeks after virus addition had ended) 43% of the number of progenitor cells that were clonable to hematopoietic colonies survived exposure to G418. This indicates that these progenitor cells had been rendered G418 resistant by virtue of containing the G418 resistance gene transferred by the retrovirus to stem cells present in the culture during the initial 14 day infection period. The rapidly perfused cultures not supplemented with HGFs had an average 12% progenitor cell survival in high [G418] between weeks 8 and 11. At culture termination at 11 weeks, the stromal layer of the IL-3+GM-CSF supplemented cultures was trypsinized and 17% of progenitor cells that were adherent to the stroma survived in high [G418]. This suggests that a significant percentage of adherent progenitor cells were also infected with the SAX virus.

Analysis of retroviral infection in LTBMCs co-cultured with irradiated SAX virus packaging cells The percentage of progenitor cells surviving in G418 when co-cultured with irradiated SAX cells varied between 0 and 36%. The cultures not supplemented with HGFs produced CFU-GM that survived in high [G418] only between weeks 4-7. After week 7, no CFU-GM were produced in these cultures that survived in high [G418], suggesting that little or no infection of stem cells occurred. The LTBMCs supplemented with IL-3+GM-CSF and co-cultured with irradiated SAX cells for 2.5 weeks produced high percentages of CFU-GM that survived in 0.8 mg/ml G418 at weeks 4, 5 and 8. However, at week 10, these cultures failed to produce CFU-GM that were resistant to G418. This suggests that little or no infection of stem cells occurred in these cultures or that the stem cells may have differentiated or died.

Retroviral infection using DC-29 retrovirus

Kinetics of cell production in LTBMCs infected with DC-29 retrovirus The number of cells produced in the cultures infected with DC-29 retroviral supernatant was HGF dependent. The cultures supplemented with IL-3+GM-CSF+Epo produced between 1.5-4 x $10^6$ cells on a weekly basis throughout the 10 weeks of culture. The cultures supplemented with IL-3+GM-CSF+Epo+MGF were more prolific, while the cultures supplemented with Hybrikine+Epo resulted in the highest cell production. Interestingly, the control cultures supplemented with IL-3+GM-CSF+Epo but not receiving DC-29 retroviral supernatant additions were less prolific than the similar cultures receiving the DC-29 retroviral supernatant. Cell production in the IL-3+GM-CSF+Epo, IL-3+GM-CSF+Epo+MGF and Hybrikine+Epo cultures (with virus addition) was significantly higher than the control culture (IL-3+GM-CSF+Epo, no virus addition) at the 5%, 1% and 1% level of significance, respectively. A part of the increased production in the cultures with retroviral supernatant addition may be due to the presence of a growth factor(s) such as MGF (*c-kit* ligand) which is known to be produced by the 3T3-based packaging cell line.

Analysis of retroviral infection in LTBMCs with supernatant DC-29 virus addition The efficiency of retroviral infection was assessed by CFU-GM survival in 1.6 mg/ml G418, a concentration that killed all bone marrow cells prior to retroviral infection. The average percent of CFU-GM surviving at week 8 (6 weeks after infection) was high in all infected cultures, Table 5.

**Table 5:** Percent of CFU-GM surviving in 1.6 mg/ml G418 after infection with DC-29 retrovirus as a function of HGF supplementation. The cultures were infected with DC-29 retroviral containing supernatent for the initial 2 weeks in culture.

| week | IL-3+GM-CSF+Epo with virus | | | | | IL-3+GM-CSF+Epo+HGF with virus | | | | | Hybrikine+Epo with virus | | | | |
|---|---|---|---|---|---|---|---|---|---|---|---|---|---|---|---|
| | 1 | 2 | 3 | ±dev. | ave. | 1 | 2 | 3 | ±dev. | ave. | 1 | 2 | 3 | ±dev. | ave. |
| 2 | 4.70 | 2.40 | 5.60 | 1.6 | 4.2 | 3.70 | 21.6 | 19.5 | 9.7 | 1.49 | 9.00 | 6.20 | 18.4 | 6.4 | 11.2 |
| 4 | 2.5 | 0.0 | 4.6 | 2.5 | 2.4 | 8.1 | 6.0 | 16.1 | 3.3 | 10.1 | 9.0 | 6.2 | 18.4 | 5.4 | 4.4 |
| 6 | 0.0 | 0.0 | 0.0 | 0.0 | 0.0 | 1.4 | 1.7 | 4.0 | 1.4 | 2.4 | 0.0 | 0.0 | 0.0 | 0.0 | 0.0 |
| 8 | 0.0 | 22.2 | * | 12.8 | 7.4 | 0.0 | 14.5 | 11.5 | 3.6 | 9.0 | 21.6 | 14.7 | 10.7 | 5.5 | 15.7 |
| 10 | * | 0.0 | * | 0.0 | 0.0 | 8.2 | 0.0 | 0.0 | 4.7 | 2.7 | 0.0 | 0.0 | 0.0 | 0.0 | 0.0 |
| 10† | 2.6 | 0.0 | * | 1.5 | 0.9 | 7.7 | 0.0 | * | 4.4 | 2.6 | 0.0 | 0.0 | 0.0 | 0.0 | 0.0 |

* N/A because no CFU-GM were counted in 0.0 mg/ml G418.

† % CFU-GM surviving in 1.6 mg/ml G418 from the trypsinized stroma in each culture at experiment termination

% survival was calculated as the (number of CFU-GM at 1.6 mg/ml G418 - ave. number of CFU-GM at 1.6 mg/ml G418 in uninfected cultures) divided by (number of CFU-GM at 0.0 mg/ml G418) * 100

EP 0 575 350 B1

Addition of MGF to the combination of IL-3+GM-CSF+Epo appears to have increased infection efficiency at weeks 8-10 in culture, with one of the cultures containing 7.7% G418 resistant colonies. The data at week 8 suggests that the cultures supplemented with Hybrikine+Epo had a high infection efficiency, although the data at week 10 suggests no infection. At week 10, note that the number of CFU-GM removed from several cultures decreased to where few or no CFU-GM would be expected be seen at approximately 10% infection. In addition, 1.5 mg/ml G418 is an extremely high antibiotic dose, enough to likely overwhelm the efficacy of the G418 resistance gene transfected at even slightly suboptimal levels. Thus, the efficiency of gene transfer into the hematopoietic stem cells in these cultures was at least 7.7% in some samples, and perhaps as high as 45% or higher.

Kinetics of nonadherent progenitor cell production

Because analysis of retroviral infection in these experiments depends on assaying for progenitor cells to infer stem cell existence, infection, and cycling, it is important to determine the effect of HGFs on progenitor cell production in culture. Also, in this retroviral experiment, MGF and Hybrikine are being used in combination with other HGFs. Both MGF and Hybrikine have not been used in rapidly perfused HGF supplemented LTBMCs, and it is therefore necessary to determine their effects on hematopoiesis.

Progenitor cell production was strongly dependent on HGF supplementation. The number of CFU-GM removed from the cultures is shown in Table 6.

Table 6:

| The cumulative number of progenitor cells removed in DC-29, HGF supplemented rapidly perfused human long-term bone marrow cultures. | | |
|---|---|---|
| Culture | Virus Added | Total Number of CFU-GM Removed |
| Innoculum | N/A | 900 |
| Control (IL-3+GM-CSF+Epo) | No | 1300 |
| IL-3+GM-CSF+Epo | Yes | 2800 |
| IL-3+GM-CSF+Epo+MGF | Yes | 5400 |
| Hybrikine+Epo | Yes | 5000 |
| Every second weekly sampling from the cultures was assayed for CFU-GM and the non assayed values were estimated by linear interpolation between two known data points. The known and interpolated values were summed to approximate the total number of CFU-GM removed from culture. | | |

Addition of retroviral supernatant increased progenitor cell removal 2.2-fold over no addition of retroviral supernatant. The increase in the number of CFU-GM removed in the cultures supplemented with retroviral supernatant and IL-3+GM-CSF+Epo+MGF or Hybrikine+Epo was 4.2 and 3.8-fold greater then the uninfected control, respectively. Removal of CFU-GM was statistically greater, at the 1% level of significance, in all viral supplemented cultures when compared to the number of CFU-GM inoculated.

Progenitor cells production in culture

A population balance on the CFU-GM compartment shows that the addition of MGF or Hybrikine to cultures supplemented with IL-3+GM-CSF+Epo have a significant positive effect on the CFU-GM pool. The addition of MGF to the combination of IL-3+GM-CSF+Epo increased CFU-GM removal 1.9-fold and CFU-GM differentiation 0.5-fold compared to similar cultures not supplemented with MGF, Table 7.

Table 7:

| The cumulative production of CFU-GM removed and differentiated in growth factor supplemented DC-29 retroviral infected rapidly perfused human long-term bone marrow cultures. | | | | |
|---|---|---|---|---|
| Culture | Number of CFU-GM removed (plated) | Number of GM cells removed ($\times 10^{-6}$) | Estimated number of CFU-GM that differentiated | Total number of CFU-GM |
| Innoculum | (900) | (0) | (0) | (900) |
| control | 1300 | 1.1 | 1100 | 2400 |

Table 7: (continued)

| The cumulative production of CFU-GM removed and differentiated in growth factor supplemented DC-29 retroviral infected rapidly perfused human long-term bone marrow cultures. | | | | |
|---|---|---|---|---|
| Culture | Number of CFU-GM removed (plated) | Number of GM cells removed ($\times 10^{-6}$) | Estimated number of CFU-GM that differentiated | Total number of CFU-GM |
| (IL-3+GM-CSF+Epo, no virus added) | | | | |
| IL-3+GM-CSF+Epo (virus added) | 2800 | 1.8 | 1800 | 4600 |
| IL-3+GM-CSF+Epo+MGF (virus added) | 5400 | 3.1 | 3100 | 8400 |
| Hybrikine+Epo (virus added) | 5000 | 6.1 | 6100 | 11,100 |
| Every second weekly sampling from the cultures was assayed for CFU-GM and the non assayed values were estimated by linear interpolation between two known data points. The known and interpolated values were summed to approximate the total number of CFU-GM removed from culture. | | | | |

The combination of Hybrikine+Epo had a very pronounced effect on CFU-GM production and differentiation. Hybrikine+Epo induced a 1.8-fold increase in CFU-GM removal and over a 3-fold increase in CFU-GM differentiation compared to the previous optimum cultures supplemented with IL-3+GM-CSF+Epo. Also, Hybrikine+Epo induced production of almost twice the number of granulocytes and macrophages than did the combination of IL-3+GM-CSF+Epo+MGF. This indicates that Hybrikine is a potent inducer of the granulocyte macrophage lineages.

Analysis of neutrophils produced from stem cells infected with CD18 encoding retrovirus CD18 deficient bone marrow was enriched for early hematopoietic cells as described above, and then cultured for 14 days with 50% daily medium exchange supplemented with 1.0 ng/ml/day GM-CSF and 1.0 ng/ml/day IL-3 and 40 U/ml/day IL1$\alpha$, and with CD18 retroviral producer line supernatant. From day 15 on, the cells were cultured under the same conditions without addition of retroviral supernatant. Nonadherent cells were removed from the cultures weekly and analyzed for the presence of cell surface CD18 by flow cytometry using a biotinylated anti-CD18 monoclonal antibody by standard methods (Updyke et al Meth. Enzymol. (1986) 121:717-725). Whereas CD18 deficient bone marrow cells failed to express any cell surface CD18 protein by this assay, neutrophils and monocytes that arose from the retrovirally infected cultures did express cell surface CD18. In triplicate cultures, expression of cell surface CD18 was 3.5%/5%/2% at 6 weeks and 11%/28%/3% at 11 weeks. Since the neutrophils and monocytes present in the cultures at 11 weeks arose form CFU-GM progenitor cells only 10-14 days earlier, these data indicate that human hematopoietic stem cells were successfully and stably transfected with the recombinant retrovirus during the first two weeks of the culture.

In interpreting the present results it is important to recognize that although several groups have demonstrated retroviral-mediated gene transfer into human hematopoietic progenitor cells, gene transfer into human hematopoietic stem cells has not been shown. The rapid decay of cell production in traditional, slowly perfused human LTBMCs limited infection determination due to the absence of progenitor cells required for the assay.

Retroviral infection in the present studies was initially assessed by growing cells removed from the LTBMCs in methylcellulose in the presence of the mammalian cell antibiotic G418. Progenitor cells infected with the SAX or DC-29 retroviruses and expressing the NEO product will be able to survive and form colonies in high concentrations of G418, whereas noninfected cells will die in high concentrations of G418. Also, production of progenitor cells that survive in high concentrations of G418, six or more weeks after virus addition has ended, requires those cells to have recently differentiated from a more primitive cell (a stem cell) and therefore suggests that stem cells were infected. Similarly, the CD18 expressed on the surface of neutrophils and monocytes produced during the 11th week of culture requires that a primitive hematopoietic stem cell was infected during the first 2 weeks of the cultures, because all mature cells, precursors, and clonogenic progenitors present during the infection period had died by 4-5 weeks in the culture.

The analysis for retroviral infection used here can underestimate the percentage of cells infected with the retrovirus due to insufficient expression of the NEO gene product. Under-expression of the transferred gene product has been shown to be a problem in human and primate models. Therefore, the percentage of progenitor cells infected in this study is probably a conservative estimate.

The percentage of progenitor cells that survived in high [G418] was approximately 40% at week 10 in the cultures infected SAX retrovirus supernatant and supplemented with IL-3+GM-CSF±IL-1$\alpha$. These initial results show that a high percentage of stem cells were infected in the cultures supplemented with retroviral supernatant during the first two weeks in culture.

The percentage of progenitor cells infected with the DC-29 retrovirus was high (0-21%) in the IL-3+GM-CSF+Epo+MGF and Hybrikine+Epo cultures during the initial 4 weeks after viral addition had ended. This high level of progenitor cell infection was probably due to direct infection of progenitor and primitive cells by the retrovirus. The percentage of progenitor cells surviving in high G418 concentration decreased 4 weeks after viral addition had ended, but rebounded 2 weeks later to 0-22% survival in high G418 concentration.

The production of G418 resistant progenitor cells in the DC-29 experiment may underestimate the percentage of progenitor cell actually infected with the DC-29 retrovirus. The high concentration of G418 used to select for infected colonies (1.6 mg/ml of G418) is twice as high as that used for the SAX infection experiment and requires high expression levels of the NEO gene product, neomycin phosphotransferase, to survive.

Interestingly, in the LTBMCs supplemented with either the SAX or DC-29 retrovirus containing supernatant, the HGFs IL-3+GM-CSF±Epo, IL-1α, or MGF or the combination of Hybrikine+Epo, the percent of progenitor cells surviving in G418 increased 6-8 weeks after viral addition ended. Although the mechanism for the increase in the percentage of G418 surviving progenitor cells towards the latter stages of the culture are not known one possibility is that stem cells that were infected during the first two weeks in culture became more active as the culture progressed. This would effectively increase the percentage of cells surviving in G418. Another possibility is that expression of the NEO gene may have increased in progenitor cells produced late in culture due to differentiation from a stem cell which had a different and better expressible integration site than did progenitor cells transfected directly during the initial infection culture period. Therefore, the high level of progenitor cell survival late in culture although possibly due to several causes, does strongly suggest that stem cells were infected in these LTBMCs.

In sum, these data document that under the culture conditions disclosed in the present invention, hematopoietic stem cells were proliferating in the cultures, permitting the incorporation of retrovirally transferred genetic material into these cells. Progenitors were continuously and actively produced from these stem cells, and these many of these progenitors contained and expressed the transfected genes. These data indicate that genetically modified human hematopoietic stem cells were present and proliferating in these cultures.

## IV. EXPERIMENTAL

### I. Formation of Transformants

The growth factor human GM-CSF (Wong, Science (1984) 228:810-815) was inserted into a eukaryotic expression vector. The hGM-CSF cDNA (EcoRI to AhaIII, approximately 700 bp fragment) was cloned into an EcoRI to PstI fragment of pSP65. (Melton, Nucl. Acids Res. (1984) 2:7035-7056). The resulting plasmid was SP65GM-CSF. The mouse metallothionein promoter (Glanville, Nature, (1981) 292:267-269) was digested with EcoRI and BglII and the approximately 2 kb fragment containing the promoter was inserted into the EcoRI to BamHI fragment of pSP65 to make p65MT. The plasmid pMT GM-CSF was then constructed by digesting pSP65GM-CSF with EcoRI, filling in the overhang with the Klenow fragment of DNA polymerase I and then digesting the resulting linearized DNA with HindIII to isolate the 700 bp fragment comprising the coding region of GM-CSF. This fragment was subcloned into the SalI filled/HindIII site of p65MT. The 2.7 kb fragment comprising the metallothionein promoter and the GM-CSF coding region was then isolated and placed into pSV2neo (Southern and Berg, J. Mol. Appl. Genet (1982) 1:327) from which the SV-40 promoter was removed. This results in the SV-40 poly A signal downstream of the GM-CSF coding sequence.

The neomycin resistant gene, which confers resistance to the antibiotic gentamicin (G418) was taken from pSV2neo by isolating the approximately 3 kb PvuII to EcoRI fragment and placing EcoRI linkers onto the PvuII site. The neo resistance gene with EcoRI ends was subcloned into the EcoRI site of the GM-CSF expression plasmid to create the plasmid MTGM-CSFneo.

The plasmid MTGM-CSFneo alone and as a cotransfection with the plasmid (Yang, Cell (1986) 47:3-10) encoding the gibbon ape IL-3 gene under the control of the SV-40 promoter and poly A site, were transfected by electroporation of linearized DNA into the African green monkey cell line CV1 and the mouse cell line NIH 3T3 cells. Transformants were selected by selection in media containing 500 mg/ml of G418, isolated, and screened for production of GM-CSF or IL-3 by bioassay of supernatants using AML-193 cells (Adams et al, Leukemia (1989) 3:314). Several of the positive lines were then employed as stroma for human bone marrow cells in Dexter culture.

In addition, normal mouse bone marrow cells were transfected with the above plasmids using the calcium/phosphate method of Okayama (Chen, Mol. Cell. Biol. (1987) 7:2745-2752) and were found to efficiently express the introduced genes.

GM-CSF and IL-3 secretion by the transfected fibroblasts was investigated. Serum free 72 hour culture supernatants were obtained from the NIH-3T3 cells and assayed for hGF secretion by $^3$H uptake on target cells inhibitable by neutralizing rabbit anti-GM-CSF or anti-IL-3 antibodies. Proliferation induced by 20 mg/ml GM-CSF was set as 100 units GM-CSF and that induced by 10 ng/ml IL-3 was set as 100 units IL-3. The co-transfected cells produced about 35 units/ml of GM-CSF and about 57 units/ml of IL-3.

II. Perfusion Chamber

The perfusion chamber is a glass cylinder with Delrin caps to allow for autoclaving without deformation and biocompatability. The caps have cylindrical groves into which the glass cylinder fits. At the bottom of the grove an O-ring is placed to seal the lumen of the chamber. The caps have several holes into which Luer (Luer Lok) fittings are provided into which media andgas delivery lines are put as well as an extended tube into the central section of the chamber to sample adherent and/or non-adherent cells. The caps are attached with three long bolts, spaced 120°, placed outside the glass cylinder; wing nuts and washers are used to tighten the assembly.

The chamber is hooked to a side reservoir. The loop contains a pump, a chamber of on-line sensors, oxygenator, and sample and injection ports in addition to the side media reservoir. The media in the side reservoir is then slowly exchanged using a separate pump. This configuration allows for separate control of the media exchange rate and the flow rate through the oxygenator and perfusion chamber. The former is used to control the longer term change in the media composition and perfusion, while the latter may be used to control the dissolved oxygen tension and flow patterns in the chamber. The use of a small mesh polysulfonate membrane allows for plug flow in the chamber and the precise control of delivery of growth factors and other special compounds which one may wish to introduce into the bioreactor in very precise amounts.

The transfected stromal cells are seeded either over a bed of shredded collagen sponge or the stromal cells are placed on one side of a 5μ porous polycarbonate filter precoated with collagen and the stromal cells allowed to adhere to the filter over a number of hours. The cells are allowed to grow in an appropriate nutrient medium until the cells become confluent on one side while sending cytoplasmic projections through the pores. Bone marrow cells are then seeded on the other side of the membrane and the stem cells attach to the intruded cytoplasmic projections which have passed through the pores.

After autoclaving the chamber and components of the loop, the reactor is assembled in a sterile environment. The media is then circulated through the side loop and chamber for a few days while signs of contamination are monitored. The central section of the bioreactor is then inoculated with either the extra-cellular matrix alone or a preinoculated extracellular matrix support that contains the stromal cells. The stromal cells may then be kept in the chamber for a period of a few days while their metabolic performance and/or growth factor responsiveness is monitored and if results are satisfactory, the bone marrow is inoculated or immediately seeded with bone marrow. In either case, the cell layer is kept at the bottom of the central section of the perfusion chamber.

The cells lay down additional extra-cellular matrix and the cell layer adheres to the support. Where the membrane is used, the chamber may be inverted and the cell layer is then located at the ceiling of the central section. In this configuration, the maturing cells settle on the bottom of the central chamber asthey loose their adherence to the stromal layer. The non-adherent cells are then harvested by constant cell flow, driven by the medium perfusion pressure, into the exit tubing.

In a typical run, the chamber was inoculated with NIH-3T3 cells on day one on shredded collagen sponge support. For the first 40 days perfusion rates and other operating variables were adjusted. At day 40 a reasonable steady state was achieved which was maintained for about 20 days. On day 64 the chamber was seeded with 33 x $10^6$ human bone marrow cells. For the first 10 days the harvested cell count decreased until it settled in a steady state of about 7-8 x $10^5$ cells produced every three days. Flow cytometric analysis showed that a constant fraction, about 20% of the harvested cells were HLA-DR positive. On day 90 a pump failure was experienced and the pH dropped below 6.9 overnight. When the perfusion rate was restored the production of non-adherent cells recovered and was approaching the previous steady state production rate when a bacterial contamination occurred. At this point, the study was terminated.

The above results demonstrated that a perfusion chamber is capable of performing ex vivo hematopoiesis, hematopoiesis may be restored ex vivo after a pH drop, the glucose concentration data showed that the hematopoietic cells grow primarily aerobically on glucose, since the glucose concentration drops after inoculation without increasing the lactate concentration indicating that oxygenation is limiting. The glucose/lactate (anaerobic) metabolism appears to be primarily due to the NIH-3T3 stromal bed. Similarly, the glutamine and ammonia concentrations reach preinoculum levels once the hematopoietic cell number levels off, implying that the glutamine consumption by the bone marrow cells is much less than that of the stromal bed.

III. Monitoring of Metabolic Products

The consumption and formation rates of glucose and lactate as well as glutamine and ammonia were determined for transfected NIH-3T3 cells. (The medium was IMDM plus 20% FCS). Increased glucose consumption was only observed for daily fed T-flasks, whereas all less frequently fed cultures follow the same slowly diminishing glucose uptake rate pattern. Cultures that were exchanged 50% daily were switched to the 100% daily exchange schedule on day 18, which resulted in an immediate increase in glucose consumption following the same trend as that observed for cultures exchanged 100% daily from day one. Lactate production rates follow a similar pattern, as the lactate yield on glucose is essentially a constant (0.9 lactate/glucose; indicating a largely anaerobic stromal metabolism).

The glutamine and ammonia concentrations show a pattern analogous to the glucose/lactate metabolism. Using values corrected for chemical decomposition of glutamine at 37°C, the glutamine consumption rate versus the glucose consumption rate shows relative uptake rates are constant, about 1:8 glutamine: glucose. The predicted optimum ratio varies with oxygen uptake rate - the ratio drops with increasing optimum uptake rate.

Analogous conclusions were supported by glucose/lactate metabolic data derived from normal bone marrow stromal fibroblasts. Under conditions of infrequent medium exchange the cultures were primarily anaerobic, with high steady state levels of lactate rapidly achieved and maintained. With more frequent medium exchange, the cell metabolism became more rapid, with increased glucose consumption and lactate production. No detectable consumption of glutamine was observed after correcting the data for spontaneous chemical decomposition. For both 3T3 cells and normal human bone marrow cells, the cells continue to divide and crowd when the serum/media exchange rate was above what appears to be a critical replacement schedule.

To further ascertain the relative importance of perfusion rate of serum versus that of nutrients, the following experiments were performed: 1) one set of T-flasks with 20% serum containing media exchanged daily; 2) two sets of T-flasks, one with 20% serum and the media exchanged every other day and one with 10% serum with the media exchanged daily; 3) two sets of T-flasks, one with 10% serum and the media exchanged every other day, one with 5% serum with the media exchanged daily; 4) two sets of T-flasks, one with 5% serum and the media exchanged every other day and one with 2.5% serum with the media exchanged daily. The serum exchange rate is the same within each group while the exchange rate of the nutrient containing media varies. The results from these experiments show that it is the exchange rate of the serum that is critical. While for the experiment 1) glucose consumption increased and by day four had substantially flattened out to a rate of about 9.5 mmoles/per day, in all of the other cases, the glucose consumption started below the original glucose consumption of Group I and dropped off in a substantially linear manner regardless of whether twice the amount of serum was used and changed every other day or half the amount of serum was used and the media changed every day. This supports the need for a critical perfusion rate of serum or one or more serum components that influence the metabolic growth behavior of the stromal cells.

It is evident from the above results, that one may grow hematopoietic cells in a bioreactor in an efficient manner. Stromal cells can be provided from homologous or heterologous sources, where the stromal cells have been transfected with genes to provide for the important growth factors. In this manner, serum need not be added to the media to support the growth of the cells. By providing for stromal cells which adhere to a support in a manner which allows for separation of hematopoietic cells from the stromal cells, the hematopoietic cells may be continuously harvested for use. By appropriate choice of combinations of factors, specific lineages of hematopoietic cells may be grown. In addition, if desired, the stromal cells may provide for a reservoir of transfecting viruses for the introduction of genes into the hematopoietic cells.

All publications and patent applications cited in this specification are herein incorporated by reference as if each individual publication or patent application were specifically and individually indicated to be incorporated by reference.

Obviously, numerous modifications and variations of the present invention are possible in light of the above teachings. It is therefore to be understood that within the scope of the appended claims, the invention may be practiced otherwise than as specifically described herein.

## Claims

1. A method for obtaining ex vivo human stem cell division comprising culturing a human stem cell composition in a liquid culture medium, wherein the culture medium is replaced at a rate of 50 to 100% daily replacement for a cell density of from $1 \times 10^4$ to $1 \times 10^7$ cells per ml of culture, while maintaining said culture under physiologically acceptable conditions.

2. A method for obtaining ex vivo human stem cell division comprising culturing a human stem cell composition in a liquid culture medium, wherein the culture medium is replaced at a rate of at least 50% daily replacement, while maintaining said culture under physiologically acceptable conditions.

3. A method for obtaining ex vivo human stem cell division comprising culturing a human stem cell composition in a liquid culture medium, wherein the liquid culture medium is at least 50% replaced every 3.5 days, while maintaining said culture under physiologically acceptable conditions.

4. The method according to any one of claims 1 to 3, comprising culturing human stem cells found in the human hematopoietic system.

5. The method according to any one of claims 1 to 3, comprising culturing human stem cells found in human bone marrow.

6. The method according to any one of claims 1-3, comprising using a medium containing IL-3, GM-CSF, steel factor, Epo, IL-$\alpha$, IL-1$\beta$, G-CSF, basic fibroblast growth factor, IL-6, IL-7, IL-8, IL-9, IL-10, IL-11, PDGF or EGF.

7. The method according to any one of claims 1-3, comprising culturing a human hematopoietic stem cell composition.

**Patentansprüche**

1. Verfahren zum Erreichen einer ex vivo-Teilung humaner Stammzellen, umfassend die Kultivierung einer Zusammensetzung humaner Stammzellen in einem flüssigen Kulturmedium, wobei das Kulturmedium mit einer 50 bis 100%igen täglichen Austauschrate für eine Zelldichte von 1 x 10$^4$ bis 1 x 10$^7$ Zellen pro ml Kultur ausgetauscht wird, wobei die Kultur unter physiologisch verträglichen Bedingungen gehalten wird.

2. Verfahren zum Erreichen einer ex vivo-Teilung humaner Stammzellen, umfassend die Kultivierung einer Zusammensetzung humaner Stammzellen in einem flüssigen Kulturmedium, wobei das Kulturmedium mit einer wenigstens 50%igen täglichen Austauschrate ausgetauscht wird, wobei die Kultur unter physiologisch verträglichen Bedingungen gehalten wird.

3. Verfahren zum Erreichen einer ex vivo-Teilung humaner Stammzellen, umfassend die Kultivierung einer Zusammensetzung humaner Stammzellen in einem flüssigen Kulturmedium, wobei das flüssige Kulturmedium alle 3,5 Tage zu wenigstens 50% ausgetauscht wird, wobei die Kultur unter physiologisch verträglichen Bedingungen gehalten wird.

4. Verfahren nach irgendeinem der Ansprüche 1 bis 3, umfassend die Kultivierung humaner Stammzellen, die im humanen hämatopoetischen System gefunden werden.

5. Verfahren nach irgendeinem der Ansprüche 1 bis 3, umfassend die Kultivierung humaner Stammzellen, die im humanen Knochenmark gefunden werden.

6. Verfahren nach irgendeinem der Ansprüche 1 bis 3, umfassend die Verwendung eines Mediums, das IL-3, GM-CSF, Steel-Factor, Epo, IL-$\alpha$, IL-1$\beta$, G-CSF, basischen Fibroblastenwachstumsfaktor, IL-6, IL-7, IL-8, IL-9, IL-10, IL-11, PDGF oder EFG enthält.

7. Verfahren nach irgendeinem der Ansprüche 1 bis 3, umfassend die Kultivierung einer Zusammensetzung humaner hämatopoetischer Stammzellen.

**Revendications**

1. Procédé pour obtenir une division *ex vivo* de cellules souches humaines consistant à cultiver une composition de cellules souches humaines dans un milieu de culture liquide, dans lequel le milieu de culture est remplacé à un taux de remplacement quotidien de 50 à 100% pour une densité de cellules comprise entre 1x10$^4$ et 1x10$^7$ cellules par mL de culture, tout en maintenant ladite culture dans des conditions acceptables sur le plan physiologique.

2. Procédé pour obtenir une division *ex vivo* de cellules souches humaines consistant à cultiver une composition de cellules souches humaines dans un milieu de culture liquide, dans lequel le milieu de culture est remplacé à un taux de remplacement quotidien d'au moins 50%, tout en maintenant ladite culture dans des conditions acceptables sur le plan physiologique.

3. Procédé pour obtenir une division ex vivo de cellules souches humaines consistant à cultiver une composition de cellules souches humaines dans un milieu de culture liquide, dans lequel le milieu de culture liquide est remplacé à au moins 50% tous les 3,5 jours, tout en maintenant ladite culture dans des conditions acceptables sur le plan physiologique.

**4.** Procédé selon l'une quelconque des revendications 1 à 3, consistant à cultiver des cellules souches humaines trouvées dans le système hématopoïétique humain.

**5.** Procédé selon l'une quelconque des revendications 1 à 3, consistant à cultiver des cellules souches humaines trouvées dans la moelle osseuse humaine.

**6.** Procédé selon l'une quelconque des revendications 1 à 3, consistant à utiliser un milieu contenant IL-3, GM-CSF, le facteur SLF, Epo, IL-$\alpha$, IL-1$\beta$, G-CSF, un facteur de croissance de fibroblastes basiques, IL-6, IL-7, IL-8, IL-9, IL-10, IL-11, PDGF ou EGF.

**7.** Procédé selon l'une quelconque des revendications 1 à 3, consistant à cultiver une composition de cellules souches hématopoïétiques humaines.

FIG.1

FIG.2

FIG.3A

FIG.3B

FIG.3C

SHEAR STRESS PROFILE

FIG.4A

FIG.4B

EP 0 575 350 B1

FIG.5A

FIG.5B

EP 0 575 350 B1